(19) Europäisches Patentamt
European Patent Office
Office européen des brevets

(11) **EP 2 931 297 B1**

(12) **EUROPEAN PATENT SPECIFICATION**

(45) Date of publication and mention
of the grant of the patent:
**06.06.2018 Bulletin 2018/23**

(21) Application number: **13807979.3**

(22) Date of filing: **13.12.2013**

(51) Int Cl.:
*A61K 36/8962* (2006.01)    *A61K 9/00* (2006.01)
*A61K 47/32* (2006.01)    *A61K 9/70* (2006.01)
*A61L 15/24* (2006.01)    *A61L 15/44* (2006.01)
*A61L 15/58* (2006.01)

(86) International application number:
**PCT/EP2013/076584**

(87) International publication number:
**WO 2014/091007 (19.06.2014 Gazette 2014/25)**

(54) **PATCH COMPRISING AN ONION EXTRACT**

PFLASTER MIT EINEM ZWIEBELEXTRAKT

TIMBRE COMPRENANT UN EXTRAIT D'OIGNON

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **14.12.2012 US 201261737315 P**

(43) Date of publication of application:
**21.10.2015 Bulletin 2015/43**

(73) Proprietor: **Merz Pharma GmbH & Co. KGaA
60318 Frankfurt am Main (DE)**

(72) Inventors:
• **RAFFAUF, Claudia
97453 Schonungen (DE)**
• **SCHULTZ, Imke
63110 Rodgau (DE)**
• **ZINK, Helga
69433 Frankfurt (DE)**
• **SCHEPPLER, Petra
55129 Mainz (DE)**

(74) Representative: **Herzog, Fiesser & Partner
Patentanwälte PartG mbB
Dudenstrasse 46
68167 Mannheim (DE)**

(56) References cited:
**US-A1- 2006 127 437    US-A1- 2010 247 689**

• **WOKOVICH A M ET AL: "Transdermal drug
delivery system (TDDS) adhesion as a critical
safety, efficacy and quality attribute",
EUROPEAN JOURNAL OF PHARMACEUTICS
AND BIOPHARMACEUTICS, ELSEVIER SCIENCE
PUBLISHERS B.V., AMSTERDAM, NL, vol. 64, no.
1, 1 August 2006 (2006-08-01) , pages 1-8,
XP027997908, ISSN: 0939-6411, DOI:
10.1016/J.EJPB.2006.03.009 [retrieved on
2006-08-01]**
• **HOCK S TAN ET AL: "Pressure-sensitive
adhesives for transdermal drug delivery
systems", PHARMACEUTICAL SCIENCE &
TECHNOLOGY TODAY, vol. 2, no. 2, 1 February
1999 (1999-02-01), pages 60-69, XP055049803,
ISSN: 1461-5347, DOI:
10.1016/S1461-5347(99)00119-4**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

Scars are areas of fibrous tissue that replace normal skin after injury. A scar results from the biological process of wound healing in the skin and other tissues of the body. With the exception of very minor lesions, every wound (e.g. after accident, disease, or surgery) results in some degree of scarring.

Thus, a scar is an end product of a wound healing process. This end product is neither aesthetically nor functionally perfect. Unwounded dermis comprises a mechanically efficient meshwork of collagen. However, wound healing in human skin results in varying degrees of scar formation, ranging clinically from fine asymptomatic scars to problematic hypertrophic and keloid scars, which may limit function, restrict further growth. In addition they may have a poor cosmetic appearance.

Treatment options for scars range from invasive procedures and surgery such as excision or laser therapy to non-invasive management such as the application of pressure and, in particular, treatments with administration of topical agents. Moreover, scars frequently remain untreated.

Agents for treating scars are known in the art.

For example, the product Kelofibrase® is known as scar cream which comprises, as active ingredients, urea and heparin sodium (60 000 I.U.), and camphor as well as customary oil/emulsifier components. This product is reportedly useful for scar treatment, for scar contractures and keloids. Here, heparin-sodium acts, as is known, as a blood-thinning agent.

Under the name Hirudoid®forte is known a gel which comprises, as active ingredient, mucopolysaccharide polysulfuric ester (445 mg, corresponding to 40 000 units in 100 g of gel). Further ingredients are the components required for the preparation of the gel, such as isopropyl alcohol, polyacrylic acid, propylene glycol and water. Mucopolysaccharide polysulfuric esters generally have a heparinoid effect and therefore correspond to the Kelofibrase® product specified above. As well as the gel, a Hirudoid®forte ointment is also known which, as well as the abovementioned active ingredients, has a mixture of monoglycerides and diglycerides with higher fatty acids and medium-chain triglycerides etc., and isopropyl alcohol, imide urea, phenoxyethanol and water. Products of this type can be used for treatment in cases of phlebopathies, superficial phlebitides, hematomas and for loosening hard scars. The product must not be applied to damaged skin.

Under the name Hylaform® is known a gel implant which contains crosslinked hyaluronic acid which is present in an aqueous sodium-chloride-containing solution for the purpose of injection. Using such an agent, skin deformations are said to be treatable. However, acute or chronic skin disorders in the affected correction area must not be present.

Under the name Linoladiol® N is known a hydrophilic O/W cream which comprises estradiol as active ingredient in the cream base. By means of this hormone-containing preparation, as well as gynecological applications, dermatological applications, such as burns, scar treatment, atrophy of the skin, perioral dermatitis and eczema in the acute and subacute stage are also stated. In this connection, however, the application limitations known for hormone-containing products, such as side effects and/or interactions, are to be taken into consideration to a considerable degree.

Further, some products for use in treating or preventing scar formation which comprise plant extracts as active ingredients are described.

In particular, gel-like products are used for this purpose. U.S. Pat. No. 5,885,581 describes such a gel-like product which comprises 20-30% by weight of polyethylene glycol 200, 0.005-0.03% by weight of preservative, 0.05-0.2% by weight of sorbic acid, 0.5-2% by weight of allantoin, 1-3% by weight of xanthan and, if desired, perfume substances, and which is characterized by 5-15% by weight of a liquid onion extract (*Allium cepa* extract), based on an aqueous carrier in an amount of about 55-65% by weight. The product thus represents a fat- (oil) -free gel and is applied externally to damaged skin tissue, in particular scarred tissue. The product is further characterized by a pH of 4.5-5.5 and a particle size of less than 50 nm.

Under the product name Contractubex®, a gel is known which comprises an onion extract (*Allium cepa* extract). Onion extracts for the treatment of scars are also disclosed in US 2010/0247689.

The product name PC 30 V describes a liquidum which comprises horse chestnut seed dry extract and also camomile blossom dry extract in 1,3-butanediol, dexpanthenol, allantoin and odor substances. This agent is said to be useful in the treatment of skin damage, such as wound chafing of sensitive pressure points and scars by orthopedic apparatuses, and also pressure sores. An indication with regard to scars as a result of operations or other skin damage is not stated here.

WO 2011/006100 discloses corticosteroids for reducing scar formation.

US 2006/127437 discloses a composition comprising an onion extract for the treatment of scars.

DE-A 37 23 248 relates to the use of thiosulfinic acid derivatives for the treatment of inflammations. These may be obtained, inter alia, by extraction from onions and subsequent chromatography. Onion extract itself is not used here.

EP-B 429 080 relates to a preparation process for S-allylcysteine-containing products, where, for example, aqueous garlic extracts are admixed with cysteine, giving S-allylcysteine.

EP-B 364 442 relates to an oil extract from at least 3 different herbs chosen from *Euphorbia,* veronica, yarrow, fumitory, garlic, nettle and marigold. This combination is used in the form of an oil, e.g. with paraffin, against psoriasis.

EP-B EP 201 956 relates to the extraction and chromatographic fractionation of, for example, tobacco, algae, garlic, where the specific substances obtained are reportedly useful for cosmetic or therapeutic applications.

The use of pressure dressings is further known for scar treatment. Application of pressure apparently increases the activity of collagenase, which is an enzyme capable of degrading and modeling the scar tissue and is employed by the body in the equilibrium of the formation and degradation of collagen during the healing process. However, pressure dressings are often very bulky rendering them uncomfortable to the user and often inconvenient to keep in place on the affected scar tissue.

Besides, it has been discovered in recent years that the shrinkage of hypertrophic scars can be increased by applying silicone-gel plates or sheets to the scars. The exact mechanism by which the silicone-gel interacts with such scars has not been established, however. A number of products are available commercially for this purpose, for instance such products as Dow Corning Silastic Sheeting, Cica-Care (Smith & Nephew), Epi-Derm (Biodermis), Nagosil (Nagor), among others. These products have the form of molded silicone-gel sheets having a thickness of 2-4 millimeters. In treating hypertrophic scars, these sheets are placed over the scars and are worn for a relatively long period of time, often from 3-12 months, until the scars either have decreased or have regenerated. Examples of recent patents which disclose such silicone-gel sheets include U.S. Pat. Nos. 5,759,560; 5,891,076; 5,895,656 and 5,919,476. The known silicone sheets are relatively rigid and after having been placed over the scar have insufficient adhesion to remain securely in position without some form of assistance. Consequently, it is necessary to secure the sheets against the skin with the aid of securing stocking, bandage, self-adhesive tape or some like means. The sheets often trap too much moisture causing irritation on the affected area. Additionally, gel sheets of the type that utilize silicone are tacky to the touch, both on the inner body, body contacting surface and the exterior surface. Having a body contacting surface which is tacky to the touch is advantageous and desirable. However, having an exterior which is tacky to the touch is not. A disadvantage of having a tacky exterior is that articles of clothing tend to adhere to the gel sheet. This presents several problems. One problem is that often the gel sheet adheres to an article of clothing with greater force than it adheres to the skin. Thus, when the article of clothing is removed, the gel sheet is removed from the body. Another problem is that the articles of clothing would adhere to the gel sheet and prevent normal range of motion. An additional problem encountered with gel sheets which are tacky to the touch is that they tend to become soiled more quickly.

Further active substance patches have been described in the prior art, some of which operate in accordance with the reservoir principle, where the active substance is delivered, for example, by way of a matrix system or with a relatively complex multilayer structure. In particular hot melt self-adhesive compositions have been proposed for this purpose, as for example in EP-A 663 431, EP-A 452 034, EP-A 305 757, DE-A43 10 012, DE-A 42 22 334 and DE-C 42 24 325. However, in the hot melt process, active substances may decompose due to the high temperature applied in this process. Further, no patches comprising a plant extract for scar treatment were described.

The other physical treatments for scars available including surgery, X-ray therapy and cryotherapy are expensive or potentially dangerous and not normally recommended. Accordingly, while there have been physical treatments, these treatments are expensive, inconvenient to use, difficult to apply or simply have not been very effective in achieving the desired purpose.

Thus, there remains the need for advantageous agents for scar treatment.

SUMMARY OF THE INVENTION

The present invention relates to a patch comprising a layer composition, wherein the layer composition comprises at least a backing liner, a matrix layer comprising an onion extract (A), an acrylate based polymer (B) or a thermoplastic hot melt adhesive (B*), and a release liner, wherein the matrix layer is disposed between the backing liner and the release liner such that the matrix layer is contacted by the backing liner and the release liner on opposite sides thereof, wherein the release liner is releasable from the matrix layer.

The present invention further relates to a method for manufacturing a patch comprising a layer composition, wherein the layer composition comprises at least a backing liner, a matrix layer comprising an onion extract (A), an acrylate based polymer (B) or a thermoplastic hot melt adhesive (B*), and a release liner, wherein the matrix layer is disposed between the backing liner and the release liner such that the matrix layer is contacted by the backing liner and the release liner on opposite sides thereof, wherein the release liner is releasable from the matrix layer, wherein the method comprises the following steps:

(a) providing the release liner,
(b) applying a coating mass comprising an onion extract (A*) and

-- an acrylate based polymer (B), or
-- a thermoplastic hot melt adhesive composition (B**)

to the release liner,
(c) drying the release liner and the coating mass applied thereto such that the matrix layer is formed on the release layer, said matrix layer comprising an onion extract (A) and

-- an acrylate based polymer (B), or
-- a thermoplastic hot melt adhesive (B*)

(d) and covering the matrix layer by means of the backing liner.

Further, the present invention relates to a patch obtained or obtainable by the method described above as well as to a patch, as described above, for use in treating and/or preventing a scar.

Furthermore, the present invention relates to a method for treating and/or preventing scars in a subject in need thereof comprising applying to said subject a patch, as described above.

DETAILED DESCRIPTION

It has surprisingly been found that the patch, described hereinabove and below, is convenient to use, easy to apply to a subject in need thereof, remains secure in position on the subject, has good pressure sensitive adhesive properties and releases the active ingredient, i.e. the onion extract, in a controlled manner. In particular, it has been found that the patch according to the invention containing at least the onion extract as active substance displays a high level of efficacy, i.e. a relatively high rate of release, as well as a good skin compatibility coupled with good adhesion. Further, the removal is easy and without inducing any irritation to the skin of the subject or any pain. Further, the patch may be prepared in a safe manner while avoiding any decomposition of the onion extract.

Therefore, in a first aspect, the present invention concerns a patch comprising a layer composition, wherein the layer composition comprises at least a backing liner, a matrix layer comprising an onion extract (A), an acrylate based polymer (B) or a thermoplastic hot melt adhesive (B*), and a release liner, wherein the matrix layer is disposed between the backing liner and the release liner such that the matrix layer is contacted by the backing liner and the release liner on opposite sides thereof, wherein the release liner is releasable from the matrix layer.

*The matrix layer:*

As described above, the matrix layer comprises an onion extract (A) and an acrylate based polymer (B), or an onion extract (A) and a thermoplastic hot melt adhesive (B*).

The Onion extract (A)

As used herein, the term "onion" refers to any *Allium* species including, but not limited to, *Allium cepa, Allium fistulosum, Allium schoenoprasum, Allium ascalonicum, Allium cernuum,* and *Allium ampeloprasum.* Thus, as used herein, the term "onion" means any type of onion including, but not limited to, any cultivated onion, any wild onion, any onion species, any intra- and inter-species onion crosses, all onion varieties, all onion genotypes and all onion cultivars.

Preferably, the extracts are obtained from the bulbs of the onion.

In particular, the onion extract (A) is an *Allium cepa* extract.

Thus, the present invention also relates to a patch, as described above, wherein the onion extract (A) is an *Allium cepa* extract. Likewise, the present invention relates to a method for preparing a patch, as described above, and a patch as described above obtainable or obtained by said method, wherein the onion extract (A) is an *Allium cepa* extract.

Preferably, the amount of the onion extract (A), more preferably the *Allium cepa* extract (A), present in the matrix layer according to the invention is in the range of from 0.01 to 5 % by weight, more preferably in the range of from 0.02 to 4 % by weight, more preferably in the range of from 0.1 to 3 % by weight, more preferably in the range of from 0.3 to 2 % by weight, more preferably in the range of from 0.5 to 1.5 % by weight, and most preferably in the range of from 0.9 to 1,1 % by weight, such as preferably 1.0 % by weight, based on the total weight of the matrix layer.

The term "onion extract (A)" as used herein means a substance or composition obtained from an onion by a process comprising the extraction, maceration or percolation of the onion material. The onion extract (A) which is present in the matrix layer refers to dry onion extract which is obtained by evaporation of the whole liquid extract to dryness.

Thus, the term "onion extract" refers to all onion components present in the liquid extract (A*) which is obtained by the process comprising the extraction, maceration or percolation of the onion material which are not removed in the evaporation process together with the respective solvent. The term "evaporation of the whole liquid extract to dryness" in this context is denoted to mean that at least 99 % by weight, more preferably at least 99.9 % by weight, preferably all of the solvent being present in the onion extract (A*) obtained by the process comprising the extraction, maceration or percolation of the onion material as described above is removed to give the onion extract (A). Solvents suitable for extraction, percolation or maceration are known to those experienced in the art. Acetone, chloroform, ethyl acetate, lower alkanols with 1 to 4 carbon atoms, alcohols or a mixture of these and water are particularly suited. Carbon dioxide in fluid or super-critical form and pressurized gases with solvent properties are also suitable as extraction agents.

Preferably the onion extract (A*) comprises a solvent $S_2$, wherein said solvent is either the solvent used for extraction, maceration or percolation of the onion material or a solvent used for redissolving the dry extract.

Preferably, $S_2$ comprises at least one alcohol and water. As to the at least one alcohol, this alcohol is preferably selected from the group consisting of ethanol, propanol, iso-propanol or mixtures thereof. Most preferably, the at least one alcohol is ethanol.

In particular, the solvent $S_2$ comprises water in an amount in the range of from 95 to 75 % by weight, preferably in the range of from 90 to 84 % by weight, and most preferably in the range of from 92 to 82 % by weight, based on the total weight of the solvent $S_2$. As described above, the solvent $S_2$ comprises the at least one alcohol, wherein said at least one alcohol is preferably present in the range of from 5 to 25 % by weight, preferably in the range of from 8 to 18 % by weight, and most preferably in the range of from 10 to 16 % by weight, based on the total weight of the solvent $S_2$.

Preferably $S_2$ comprises ethanol in an amount in the range of from 5 to 25 % by weight and water in an amount of from 95 to 75 % by weight, more preferably ethanol in an amount in the range of from 8 to 18 % by weight and water in an amount of from 92 to 82 % by weight, and most preferably ethanol in an amount in the range of from 10 to 16 % by weight and water in an amount of from 90 to 84 % by weight.

Thus, the present invention also relates to a method for manufacturing a patch, as described above, as well as to a patch obtained or obtainable by said method, wherein the onion extract (A*) comprises a solvent $S_2$, wherein $S_2$ comprises ethanol in an amount in the range of from 5 to 25 % by weight and water in an amount of from 95 to 75 % by weight, based on the total weight of the solvent $S_2$.

$S_2$ may optionally comprise further organic solvents such as, for example, isopropanol or methanol. Preferably, $S_2$ comprises less than 0.1 % by weight of further organic solvents in total, preferably less than 0.05 % by weight, based on the total weight of the solvent $S_2$. As to the amount of onion components present in the onion extract, these components are preferably present in an amount of 20 to 33 % by weight, preferably 25 to 30 by weight, based on the total amount the onion extract (A*), wherein this amount refers to the weight of the dry onion extract, before dissolving this dry onion extract in the Solvent $S_2$.

According to a preferred embodiment of the invention, the onion extract (A*) is an onion extract which is obtained or obtainable by a process comprising an extraction, maceration or percolation of the onion material, either of fresh or dried onions, with a suitable solvent $S_1$ and a subsequent partial or preferably complete removal of the solvent. Preferably, after complete removal of the solvent, the remaining residue is redissolved in at least one suitable solvent, preferably in the above described solvent $S_2$. Optionally, the process for preparing the onion extract (A*) may comprise further steps, such as purification steps, in particular filtration steps.

As to the solvent used in the process to obtain the onion extract described above, any suitable solvent may be used. This solvent is referred to hereinunder as solvent $S_1$. $S_1$ may be the same or may differ from the above described solvent $S_2$. Preferably, $S_1$ comprises at least one alcohol. As to the at least one alcohol, this alcohol is preferably selected from the group consisting of ethanol, propanol, iso-propanol or mixtures thereof. Most preferably, the at least one alcohol comprised in $S_1$ is ethanol.

Preferably, the at least one alcohol is present in an amount of at least 70 % by weight, preferably of at least 80 % by weight, more preferably of at least 90 % by weight, and most preferably of at least 96 % by weight, based on the total weight of the solvent $S_1$.

$S_1$ may optionally comprise water and/or further organic solvents such as, for example, isopropanol or methanol. Preferably, $S_1$ comprises less than 0.1 % by weight of further organic solvents in total, preferably less than 0.05 % by weight based on the total weight of the solvent $S_1$. In particular, $S_1$ comprises water in an amount of less than 30 % by weight, preferably of less than 20 % by weight, more preferably of less than 10 % by weight, and most preferably of less than 4 % by weight.

According to an alternative embodiment of the invention, $S_1$ comprises ethanol in an amount in the range of from 5 to 25 % by weight and water in an amount of from 95 to 75 % by weight, more preferably ethanol in an amount in the range of from 8 to 18 % by weight and water in an amount of from 92 to 82 % by weight, and most preferably ethanol in an amount in the range of from 10 to 16 % by weight and water in an amount of from 90 to 84 % by weight.

Preferably, fresh or dried onions or parts of onions, such as onion chips, are extracted with a solvent $S_1$, as described above, comprising at least one alcohol to give a liquid phase $L_1$ and a solid residue $R_o$, or a composition consisting of the liquid phase $L_1$ and the solid residue $R_0$.

In particular, the ratio of amount of onion (weight) to solvent $S_1$ (weight) is in the range of from 0.5 : 0.9 to 0.7 : 1, more preferably in the range of from about 0.65 : 1.

The extraction can be carried out in one or more extraction steps. Preferably a multi-stage extraction is carried out in which a multiplicity of separating stages connected in series is used.

After the extraction, described above, the liquid phase ($L_1$) is preferably separated from the solid residue ($R_o$).

Between the separation step and the extraction step, the liquid phase $L_1$ and the solid residue $R_o$ are preferably allowed to stand for a time in the range of from 1 h to 4 weeks, preferably in the range of from 1 day to 3 weeks, more preferably about 1 to 2 weeks, in particular at a temperature in the range of from 10 to 40 °C, more preferably at a temperature in the range of from 10 to 30 °C, most preferably at room temperature. Thereby, further solid residue $R_o$ may precipitate from the liquid phase $L_1$.

The separation step may be carried out by any suitable method known to those skilled in the art. According to one embodiment of the invention, the separation is carried out by filtration. The term "filtration" or "filtering" refers to the process of removing essentially all, preferably all, of the solid residue $R_o$, which may be present as suspended particles, from the liquid phase by passing the composition through one or more membranes or filters.

The liquid phase $L_1$ obtained is subsequently, optional after further purification steps, concentrated, preferably evaporated to dryness as mentioned above, to give residue $R_1$. Optionally, the liquid phase $L_1$ obtained is allowed to stand for a time in the range of from 1 h to 4 weeks, preferably in the range of from 1 day to 3 weeks, more preferably about 1 to 2 weeks, in particular at a temperature in the range of from 10 to 40 °C, more preferably at a temperature in the range of from 10 to 30 °C, most preferably at room temperature. Thereby, further solid residue may precipitate from the liquid phase $L_1$. In case further precipitates are formed, preferably at least one filtration is carried out.

In the preferred case in which $L_1$ is evaporated to dryness, the residue $R_1$ corresponds to the dry onion extract mentioned above.

Subsequent to the evaporating step, the method may comprise further steps, such as, e.g. at least one purification step and/or at least one homogenization step. Preferably, the residue $R_1$, more preferably the dry onion extract $R_1$, is at least homogenized.

As described above, residue $R_1$, preferably the homogenized residue $R_1$, is preferably redissolved in the above-described solvent $S_2$, with $S_2$ preferably comprising at least one alcohol and water, to give the onion extract (A*).

Preferably, the onion extract (A*) comprises the solvent $S_2$ in an amount in the range of from 67 to 80 % by weight, preferably in the range of from 70 to 75 % by weight, more preferably in the range of from 70 to 72 % by weight, based on the total weight of the onion extract (A). Further, the onion extract (A*) preferably comprises the residue $R_1$ in an amount in the range of from 20 to 33 % by weight, preferably in the range of from 25 to 30 % by weight, more preferably in the range of from 28 to 30 % by weight, based on the total weight of the onion extract (A*).

In particular, the onion extract (A*) consists of 20 to 33 % by weight of residue $R_1$ and 67 to 80 % by weight of the solvent $S_2$, preferably of 25 to 30 % by weight of residue $R_1$ and 70 to 75 % by weight of the solvent $S_2$, and most preferably of 28 to 30 % by weight of residue $R_1$ and 70 to 72 % by weight of the solvent $S_2$, based on the total amount of the onion extract (A*), with the sum of the amounts of $R_1$ and $S_2$ giving 100 % by weight.

Preferably, the onion extract (A*) is an onion extract obtained or obtainable by a process comprising the steps of

(a1) extracting fresh or dried onions, preferably *Allium cepa,* with a solvent $S_1$ comprising at least one alcohol to give a liquid phase $L_1$ and a solid residue $R_o$,
(b1) separating the liquid phase $L_1$ from the solid residue $R_o$,
(c1) evaporating the liquid phase $L_1$ to give a residue $R_1$,
(d1) redissolving the residue $R_1$ in a solvent $S_2$ comprising at least one alcohol and water to give an onion extract (A*),

wherein the onion extract (A*) comprises $R_1$ preferably in an amount of 20 to 33 % by weight, based on the total weight of the onion extract (A*).

The onion extract (A*) is then preferably applied together with at least the acrylate based polymer or the thermoplastic hot melt adhesive composition (B**) onto the release liner, and the release liner and the coating mass applied thereto are dried such that the matrix layer comprising the onion extract (A) and the acrylate based polymer (B), or the onion extract (A) and the thermoplastic hot melt adhesive (B*), is formed on the release layer. Thus, the present invention also relates to a method for manufacturing a patch and to a patch obtained or obtainable by said method, wherein the method comprises the following steps:

(a) providing the release liner,
(b) applying a coating mass comprising an onion extract (A*) and

-- an acrylate based polymer (B), or
-- a thermoplastic hot melt adhesive composition (B**)

to the release liner,
(c) drying the release liner and the coating mass applied thereto such that the matrix layer is formed on the release layer, said matrix layer comprising an onion extract (A) and

-- an acrylate based polymer (B), or
-- a thermoplastic hot melt adhesive (B*)

and covering the matrix layer by means of the backing liner, wherein the onion extract (A*) is an onion extract obtained or obtainable by a process comprising the steps

(a1) extracting fresh or dried onions, preferably *Allium cepa,* with a solvent $S_1$ comprising at least one alcohol to give a liquid phase $L_1$ and a solid residue $R_o$,
(b1) separating the liquid phase $L_1$ from the solid residue $R_o$,
(c1) evaporating the liquid phase $L_1$ to give a residue $R_1$,
(d1) redissolving the residue $R_1$ in a solvent $S_2$ comprising at least one alcohol and water to give an onion extract (A*)

The Polyacrylate Polymer

As described above, according to one embodiment of the invention, the matrix layer comprises the polymer (B), said polymer being an acrylate based polymer. The term "acrylate based polymer" refers to polymers comprising building blocks derived from acrylate monomers, thus monomers of the formula $CH_2=CH\text{-}COOR$. Typical acrylate monomers are normally alkyl acrylates with R being an alkyl group, in particular an alkyl group having of from 1 to 20 carbon atoms, preferably 4 to 10 carbons. Useful alkyl acrylates include ethyl acrylate, butyl acrylate, amyl acrylate, hexyl acrylate, 2-ethylhexyl acrylate, ethylhexyl acrylate, octyl acrylate, isooctyl acrylate, decyl acrylate, dodecyl acrylates, with 2-ethylhexyl acrylate, butyl acrylate, and iso-octyl acrylate being preferred.

Preferably the acrylate based polymer is an acrylate-vinylacetate based polymer. The term "acrylate-vinylacetate based polymer" refers to co-polymers comprising building blocks derived from the monomeric components acrylate (B1) and vinylacetate (B2).

Thus, the present invention also relates to a patch, as described above, and a patch obtainable or obtained by the above described method, wherein the matrix layer comprises acrylate-vinylacetate based polymer (B) comprising building blocks derived from the monomeric components acrylate (B1) and vinylacetate (B2). Preferably the acrylate (B1) is selected from the group consisting of 2-ethylhexyl acrylate, ethylhexyl acrylate butylacrylate or a mixture of two or more thereof.

The polymer (B) may comprise further building blocks besides the building blocks derived from acrylate (B1) and optionally vinylacetate. According to one preferred embodiment of the invention, the polymer (B) further comprises building blocks derived from acrylamides, in particular derived from t-octyl acrylamid.

Preferably, the polymer (B) further comprises building blocks derived from functional monomers which, when incorporated into the polymer (B), result in building blocks comprising carboxylic acid groups. Useful carboxylic acid monomers to provide the functional group preferably contain from about 3 to about 6 carbon atoms and include, among others, acrylic acid, methacrylic acid, itaconic acid and the like. Acrylic acid, methacrylic acid and mixtures thereof are preferred as acids. In particular, the polymer comprises acrylic acid.

According to a particularly preferred embodiment, the polymer (B) comprises, preferably essentially consists of, building blocks derived from acrylic acid, 2-ethylhexyl acrylate and vinyl acetate.

According to a further preferred embodiment, the polymer (B) comprises, preferably essentially consist of, building blocks derived from acrylic acid, ethylhexyl acrylate, butyl acrylate, t-octylacrylamide and vinyl acetate.

Preferably, the polymer (B) comprises a crosslinking agent, such as an external crosslinking agent and/or an internal crosslinking agent.

The term "internal crosslinking agent" is denoted to mean building blocks derived from polyfunctional monomers, which when incorporated into the polymer during polymerization as building block form act as crosslinker. As example, compounds having at least two non-conjugated carbon-carbon double bonds per molecule are mentioned, in particular diallyl maleate, diallyl phthalate and multifunctional acrylates and methacrylates including polyethylene glycol diacrylate, hexanediol diacrylate, ethoxylated trimethylolpropane triacrylate, pentaerythritol triacrylate, propylene glycol diacrylate and trimethylolpropane trimethacrylate.

The term "external crosslinking agent" is denoted to mean a compound which causes post polymerization crosslinking. External crosslinking agents include metal salts such as zirconium ammonium carbonate, zinc ammonium carbonate, aluminum acetate, zinc acetate and chromium acetate.

Preferably, the matrix layer comprises the acrylate based polymer (B) in an amount in the range of from 99.8 to 80 % by weight, more preferably in the range of from 99.5 to 84 % by weight, more preferably in the range of from 98.8 to 89 % by weight, more preferably in the range of from 98.4 to 92 % by weight, more preferably in the range of from 97.5 to 93,5 % by weight, and most preferably in the range of from 96 to 94 % by weight, such as preferably 95 % by weight, based on the total weight of the matrix layer.

The thermoplastic hot melt adhesive composition B** and the thermoplastic hot melt adhesive B*.

As described above, according to another embodiment of the invention, the matrix layer comprises the hot melt adhesive (B*), wherein said matrix layer is preferably obtained or obtainable by applying a coating mass comprising an onion extract (A*) and a thermoplastic hot melt adhesive composition (B**) to the release liner, and drying the release liner and the coating mass applied thereto such that the matrix layer is formed on the release layer, said matrix layer comprising an onion extract (A) and the thermoplastic hot melt adhesive (B*)

The term "thermoplastic" refers to the capability of a compound to reversibly soften or fuse when heated and harden again when cooled. Hot melt adhesives are solid adhesives which can be applied as a melt to the substrates to be joined and harden by solidification during cooling after assembly at room temperature. In thermoplastic adhesives, this process is reversible. It is based essentially on material characteristics attributable to polymers such as polyamides, polyesters, polystyrenes or polyolefins. These polymers decisively determine the adhesive properties regarding adhesion, strength and temperature characteristics. To increase adhesion usually tackifiers are added. Further, these hot melt adhesives usually comprise plasticizers to enhance flexibility and/or waxes and/or crystallization accelerators to improve the morphology. The properties of the hot melt materials can also be selectively changed by addition of similar or different polymers.

Usually the molten adhesive is sprayed, or coated as a film. Typically hot melt adhesives can be based on polymers such as polyolefins (ethylene- or propene-based polymers), or functionalized polyolefins (ethylene or propene copolymers with oxygenated function containing monomers), or styrene block copolymers containing at least one rubbery phase, like SIS, or SBS. Styrene block copolymers are of interest due to their dual characteristics, i.e. cohesion of the styrenic phase associated with the rubber behavior of another phase.

Preferably, the polymer (B*) is a hot melt thermoplastic rubber. Preferably, the hot melt adhesive composition (B**) and the hot melt adhesive (B*) comprise a styrene polymer, more preferably a styrene block co-polymer such as SIS or SBS.

Thus, the present invention also relates to a patch, as described above, wherein the matrix layer comprises a hot melt adhesive (B*), wherein said hot melt adhesive comprise a styrene polymer, more preferably a styrene block co-polymer. Further, the present invention relates to a method, as described above, comprising applying a coating mass comprising an onion extract (A*) and a thermoplastic hot melt adhesive composition (B**) to the release liner, and drying the release liner and the coating mass applied thereto such that the matrix layer is formed on the release layer, said matrix layer comprising an onion extract (A) and the thermoplastic hot melt adhesive (B*), the hot melt adhesive composition (B**) and the hot melt adhesive (B*) comprise a styrene polymer, more preferably a styrene block co-polymer.

More preferably, the hot melt adhesive composition (B**) and the hot melt adhesive (B*) additionally comprise at least on tackifying resin and/or at least one plasticizer.

The tackifying resin is preferably selected from the group consisting of aliphatic hydrocarbon resins and their hydrogenated derivatives, hydrogenated cycloaliphatic hydrocarbon resins, aromatic modified aliphatic or hydrogenated cycloaliphatic hydrocarbon resins, aliphatic modified aromatic hydrocarbon resins, partially or fully hydrogenated aromatic hydrocarbon resins, polyterpene, terpene-phenol resins, hydrogenated resin ester and styrenated polyterpene resins.

A suitable plasticizer may be selected from the group, which not only includes the usual plasticizing oils such as mineral oil but also olefin oligomers and low molecular weight polymers, glycol benzoates, as well as vegetable and animal oil and derivatives of such oils. The oligomers may be polypropylenes, liquid polybutenes, hydrogenated polyisoprene, hydrogenated butadiene or the like having average molecular weights between about 100 and about 10,000 g/mol. Suitable vegetable and animal oils include glycerol esters of the usual fatty acids and polymerization products thereof. Other plasticizers may be used provided they have suitable compatibility.

Further, the the hot melt adhesive composition B** and the hot melt adhesive B* preferably comprise additionally at least one stabilizer or antioxidant. Examples include sterically hindered phenols, phosphites or thioesters, high molecular weight hindered phenols and multifunctional phenols such as sulfur and phosphorus-containing phenols. Hindered phenols are well known to those skilled in the art and may be characterized as phenolic compounds which also contain sterically bulky radicals in close proximity to the phenolic hydroxyl group thereof. In particular, tertiary butyl groups generally are substituted onto the benzene ring in at least one of the ortho positions relative to the phenolic hydroxy group. The presence of these sterically bulky substituted radicals in the vicinity of the hydroxyl group serves to regard its stretching frequency and, correspondingly, its reactivity; this steric hindrance thus providing the phenolic compound with its stabilizing properties. Representative hindered phenols include; 1,3,5-trimethyl 2,4,6-tris(3,5-di-tert-butyl-4-hydroxybenzyl) benzene; pentaerythrityl tetrakis-3 (3,5-di-tert-butyl-4-hydroxypphenyl) propionate; n-octadecyl-3 3,5-di-tert-butyl-4-hydroxyphenyl)-propionate; 4,4'-methylenebis (2,6-tert-butylphenol); 4,4'-thiobis (6-tert-butyl-o-cresol); 2,6-di-tertbutylphenol; 6-(4-hydroxyphenoxy)-2,4-bis(n-octyl-thio) 1,3,5 triazine; di-n-octadecyl 3,5-di-tert-butyl-4-hydroxy-benzylphosphonate; 2-(n-octylthio) ethyl 3,5-di-tert-butyl-4-hydroxy-benzoate; and sorbitol.

Further additives, such as colorants such as titanium dioxide, fillers such as talc, clay or adhesion promoters may be present. Such additives are usually used in amounts of up to 5 wt.-%, preferably in amounts of from about 3 to 5 wt.-% of the hot melt adhesive. Suitable additives are known to the expert.

Suitable hot melt adhesive compositions are, e.g., Nolax M11.13, Nolax M13.320, Dermatak H447A and Dermatak H524B.

Preferably, the matrix layer comprising the thermoplastic hot melt adhesive (B*), and the onion extract (A) is obtained, by

(a2) dissolving a thermoplastic hot melt adhesive composition (B**) in a solvent $S^{hm}$,

(b2) mixing this solution with at least an onion extract (A*) to give a coating mass,

(c2) applying the coating mass onto a release liner, and

drying the release liner and the coating mass applied thereto such that the matrix layer comprising an onion extract (A) and the thermoplastic hot melt adhesive (B*) is formed on the release layer.

Preferably step (c2) is carried out at a temperature in the range of from 0 to 50 °C, more preferably, in the range of from 5 to 40 °C, more preferably at a temperature in the range of from 10 to 30 °C, more preferably at a temperature in the range of from 15 to 25 °C.

Surprisingly, it has been found that a suitable matrix comprising an onion extract may be prepared without melting the adhesive prior to its application onto the release liner but instead by dissolving or dispersing a hot melt adhesive composition in a solvent to give a coating mass and by applying this coating mass onto the release liner. This process is particularly advantageous in that the onion extract is stable under these conditions so that any decomposition of the onion extract due to too high temperatures may be avoided. Thus, preferably the thermoplastic hot melt adhesive composition B** is not melted in order to apply the coating mass onto the release liner. Instead, the thermoplastic hot melt adhesive composition B** is dissolved or dispersed in a solvent and the solution or dispersion is applied onto the release liner.

Thus, the present invention also relates to a patch comprising a layer composition, wherein the layer composition comprises at least

a) a backing liner,
b) a matrix layer comprising an onion extract (A), and

-- an acrylate based polymer (B), or
-- a thermoplastic hot melt adhesive (B*),

c) and a release liner, wherein

the matrix layer is disposed between the backing liner and the release liner such that the matrix layer is contacted by the backing liner and the release liner on opposite sides thereof, wherein the release liner is releasable from the matrix layer, and wherein in case the matrix layer comprises a thermoplastic hot melt adhesive (B*), and an onion extract (A), wherein the matrix layer is obtained, by

- dissolving or dispersing a thermoplastic hot melt adhesive composition (B**) in a solvent $S^{hm}$,
- mixing this solution or dispersion with at least an onion extract (A*) to give a coating mass,
- applying the coating mass onto a release liner, and
- drying the release liner and the coating mass applied thereto such that the matrix layer comprising an onion extract (A) and the thermoplastic hot melt adhesive (B*) is formed on the release layer, and comprising an onion extract (A*) and an thermoplastic hot melt adhesive (B*) to the release liner,

preferably wherein the hot melt adhesive composition B** is not melted prior to the application of the coating mass onto the release liner.

The solvent $S^{hm}$ is preferably an organic solvent, more preferably an organic solvent selected from the group consisting of hexane, cyclohexane, toluene, methylethylketone, ethyl acetate, heptane and mixtures of two or more thereof, more preferably ethyl acetate and/or heptane.

Emulsifier:

Preferably, the matrix layer comprises at least one emulsifier. In particular, the at least one emulsifier (i.e., emulsifying agents) is preferably used in amounts effective to provide uniform blending of ingredients present in the coating mass prior to applying said coating mass to the release liner.

Thus, the present invention also relates to a patch, as described above, and a patch obtainable or obtained by the above described method, wherein the matrix layer comprises at least one emulsifier.

Preferred emulsifiers include one or more of

(i) anionics such as fatty acid soaps, e.g., potassium stearate, sodium stearate, ammonium stearate, and triethanolamine stearate; polyol fatty acid monoesters containing fatty acid soaps, e.g., glycerol monostearate containing either potassium or sodium salt; sulfuric esters (sodium salts), e.g., sodium lauryl 5 sulfate, and sodium cetyl sulfate; and polyol fatty acid monoesters containing sulfuric esters, e.g., glyceryl monostearate containing sodium lauryl surfate; in particular

(ii) cationics such as N(stearoyl colamino formylmethyl) pyridium; N-soya-N-ethyl morpholinium ethosulfate; alkyl dimethyl benzyl ammonium chloride; diisobutylphenoxytheoxyethyl dimethyl benzyl ammonium chloride; and cetyl pyridium chloride; and

(iii) nonionics such as polyoxyethylene fatty alcohol ethers, e.g., monostearate; polyoxyethylene lauryl alcohol; polyoxypropylene fatty alcohol ethers, e.g., propoxylated oleyl alcohol; polyoxyethylene fatty acid esters, e.g., polyoxyethylene stearate; polyoxyethylene sorbitan fatty acid esters, e.g., polyoxyethylene sorbitan monostearate; sorbitan fatty acid esters, e.g., sorbitan; polyoxyethylene glycol fatty acid esters, e.g., polyoxyethylene glycol monostearate; and polyol fatty acid esters, e.g., glyceryl monostearate and propylene glycol monostearate; and ethoxylated lanolin derivatives, e.g., ethoxylated lanolins, ethoxylated lanolin alcohols and ethoxylated cholesterol, glyceryl monooleat and caprylocaproyl macrogol glyceride.

Preferably, the emulsifier is a nonionic surfactant, more preferably a surfactant selected from the group consisting of polyethyleneglycol hydroxystearate, such as polyethylene glycol (15)-hydroxystearate (such as Solutol®), glyceryl monooleate (Peceol®), a caprylocaproyl macrogol glyceride (Labrasol®), cremophor, and mixtures of two or more thereof.

Most preferably, the emulsifier is caprylocaproyl macrogol glyceride (Labrasol®).

Thus, the present invention also relates to a patch, as described above, and a patch obtainable or obtained by the above described method, wherein the matrix layer comprises caprylocaproyl macrogol glyceride as emulsifier.

Preferably, the matrix layer comprises the at least one emulsifier in an amount in the range of from 0.1 to 10 % by weight, more preferably in the range of from 0.5 to 8 % by weight, more preferably in the range of from 1 to 5 % by weight, more preferably in the range of from 1 to 4 % by weight, more preferably in the range of from 1.5 to 3.5 % by weight, and most preferably in an amount of about 3 % by weight, based on the total weight of the matrix layer.

Further active agents:

The matrix layer present in the patch of the present invention may also comprise, besides the onion extract (A), at least one further active agent, in particular agents that sooth, condition and/or heal the skin.

The contained active agent may be selected from all active agents and mixtures thereof that can be applied to the surface of the skin. The active agent can act cosmetically or pharmaceutically. The active agent can be completely of plant origin or can be synthetic. The group of active agents may overlap with other groups of further ingredients described above and below, such as the thickening agents and the emulsifiers.

The at least one active agent of the invention is preferably selected from the group of substances having moisturizing and barrier strengthening properties, such as e.g. hydroviton, an emulation of NMF, chitosan, alginat, pyrrolidone carbonic acid and salts thereof, lactic acid and salts thereof, glycerol, sorbitol, propylene glycol and urea, substances of the group of proteins and protein hydrolysates, such as e.g. collagen, elastin as well as silk protein, substances of the group of glycose aminoglucanes, such as e.g. hyaluronic acid, of the group of carbohydrates, such as e.g. pentavitin that corresponds in its composition to the carbohydrate mixture of the human subcomeus layer, and the group of lipids and lipid precursors such as for example ceramides. Further advantageous active agents in the sense of the present invention may be selected from the group of vitamins, such as e.g. panthenol, niacin, α-tocopherol and its esters, vitamin A as well as vitamin C. Moreover, active agents selected from the group of antioxidants, e.g. galates and polyphenols may be used. Urea, hyaluronic acid and pentavitin are preferred substances.

It is further preferred that substances having skin soothing and regenerative action, such as agents promoting wound healing, are employed as active agents, such as e.g. panthenol, panthenol derivatives (e.g., ethyl panthenol), hyaloronic acid, allantoin, bisabolol, dipotassium glycyrrhizinate and phytosteroles. Advantageous active agents in the sense of the present invention are also plants and plant extracts. These are e.g. algae, aloe, arnica, barber's rash, comfrey, birch, nettle, calendula, oak, ivy, witch hazel, henna, hop, camomile, ruscus, peppermint, marigold, rosemary, sage, green tea, tea tree, horsetail, thyme and walnut as well as extracts thereof.

Preferably, the matrix layer further comprises allantoin.

In particular, the matrix layer comprises at least one further active agent, in particular allantoin, in an amount in the range of from 0.01 to 5 % by weight, more preferably in the range of from 0.02 to 4 % by weight, more preferably in the range of from 0.1 to 3 % by weight, more preferably in the range of from 0.3 to 2 % by weight, more preferably in the range of from 0.5 to 1.5 % by weight, and most preferably in the range of from 0.9 to 1,1 % by weight, such as preferably 1.0 % by weight, based on the total weight of the matrix layer.

The matrix layer preferably has a thickness in a range from 20 $\mu$m to 100 $\mu$m and preferably in a range from 25 $\mu$m to 80 $\mu$m, more preferably in the range from 30 $\mu$m to 60 $\mu$m, and more preferably of 40 $\mu$m.

Thus, the present invention also relates to a patch, as described above, and a patch obtainable or obtained by the above described method, wherein the matrix layer has a thickness in a range from 20 $\mu$m to 100 $\mu$m.

The matrix layer may comprise a rectangular surface if seen in a direction of the layer composition. For example, the surface of the matrix layer may comprise a length of 12 cm and a width of 3 cm. It is to be noted that the term rectangular also encompasses slight deviations from an exact rectangular shape. For example, the surface may comprise rounded edges.

The backing liner:

As described above, the patch according to the invention comprises a backing liner.

Preferably, said backing liner is at least partially made of an occlusive material. The term "occlusive" in the context of the present invention refers to materials that are substantially oxygen and vapor impermeable and liquid impermeable. Suitable occlusive materials preferably have an moisture vapor transmission rate (MVTR) of less than 500, such as in the range of from 0 to 500, more preferably of less than 400, more preferably of less than 300, more preferably of less than 200, measured according to European testing standard EN 13726-2:2002 'Test methods for primary wound dressings, part 2: Moisture vapour transmission rate of permeable film dressings' specified as mass water (in gram), which evaporates during 24 hours through an area of 1 square meter test material.

The weighing difference of the test vessel before and after the measuring time has to be calculated according to the following equation:

$$MVTR = \frac{(W1 - W2) * Fa * 24}{t}$$

W1 = mass prior testing [g]
W2 = mass after testing [g]
t = Testing time [h]
Fa = factor to convert the test area to 1 m$^2$

In particular, the backing liner comprises films of metal foil or polymers such as polyvinylidene chloride, polyester, polyethylene terephthalate, polyurethane, polyvinyl fluoride or polyvinyl chloride, polyolefin, polyethylene, polypropylene, PTFE or nitrocellulose. Other suitable polymeric materials include water soluble polymers such as polyvinylpyrrolidones, polyvinyl-alcohols and the like.

Preferably, the polymer is a polyolefine. Suitable polyolefins are, e.g., polyethylenes (with and without acetate moieties, e.g., ethylene vinyl acetate), polypropylenes and the like.

More preferably, the backing liner is a foam liner, preferably a foam liner comprising a polyolefinic foam, more preferably a polyethylene foam.

Preferably, the backing liner fully covers a surface of the matrix layer facing the backing liner. Thus, the matrix layer is reliably protected and the occlusive effect is enhanced.

Preferably, the backing liner comprises a greater thickness than the matrix layer. This further improves the occlusive characteristics.

For example, the backing liner comprises a thickness in a range from 150 $\mu$m to 500 $\mu$m and preferably in a range from 200 $\mu$m to 400 $\mu$m, more preferably in the range from 200 $\mu$m to 400 $\mu$m and still more preferably 300 $\mu$m.

The backing liner may comprise a rectangular surface if seen in a direction of the layer composition. For example, the surface of the backing liner may comprise a length of 12 cm and a width of 3 cm. It is to be noted that the term rectangular also encompasses slight deviations from an exact rectangular shape. For example, the surface may comprise rounded edges.

Preferably, the backing liner is adhered to the matrix layer. For example, the backing liner is permanently adhered to the matrix layer. This prevents an unwanted release of the backing liner and an unwanted release of the active agents of the matrix layer.

The release liner

The release liner may comprise a rectangular surface if seen in a direction of the layer composition. For example, the surface of the backing liner may comprise a length of 13 cm and a width of 4 cm. It is to be noted that the term rectangular also encompasses slight deviations from an exact rectangular shape. For example, the surface may comprise rounded edges.

Preferably, the release liner is at least partially siliconized. For example, a top side of the release liner facing and contacting the matrix layer may be siliconized. This facilitates the release of the release liner from the matrix layer for using the patch.

Preferably, the release liner comprises a greater surface area than a surface of the matrix layer facing the release liner as mentioned above. This reliably protects the matrix layer from contamination.

For example, the release liner protrudes from the matrix layer in at least one direction being perpendicular with respect to a direction of the layer composition. More preferably, the matrix layer comprises side faces and the release liner protrudes from the side faces in a direction being perpendicular with respect to a direction of the layer composition.

Preferably, the matrix layer is disposed substantially symmetrically on the release liner. For example, the release liner may protrude from all side faces of the matrix layer at 5 mm. This reliably protects the matrix layer from contamination.

The patch has preferably a bond strength of from 0.2 to 5 N/cm, more preferably of from 0.2 to 3 N/cm, preferably 1 N/cm, measured by FTM-2 (90 ° peel test).

The active substances present in the matrix layer are preferably released with a release rate in the range of from 10 to 100 %, more preferably in the range of from 20 to 100 %, more preferably in the range of from 50 to 100 %, based on the total amount of each active ingredient present in the patch. Thus, the onion extract is preferably released with a release rate in the range of from 5 to 100 %, more preferably in the range of from 10 to 100 %, more preferably in the range of from 20 to 100 %, more preferably in the range of from 30 to 100 %, more preferably in the range of from 40 to 100 %, more preferably in the range of from 50 to 100 %, based on the total amount of the onion extract present in the patch. Further, allantoin is preferably released with a release rate in the range of from 10 to 100 %, more preferably in the range of from 20 to 100 %, more preferably in the range of from 50 to 100 %, based on the total amount of allantoin present in the patch. The active ingredients are thereby preferably released over a time range of from 4 to 8 h, preferably of from 5 to 7 h, preferably over around 6 h.

Method for manufacturing a patch

As described above, the present invention also relates to a method for manufacturing a patch, and to a patch obtained or obtainable by said method, wherein the method comprises the following steps:

a) providing the release liner,
b) applying a coating mass comprising an onion extract (A*) and

-- an acrylate based polymer (B), or
-- a thermoplastic hot melt adhesive composition (B**)

to the release liner,
c) drying the release liner and the coating mass applied thereto such that the matrix layer is formed on the release layer, said matrix layer comprising an onion extract (A) and

-- an acrylate based polymer (B), or
-- a thermoplastic hot melt adhesive (B*)

d) and covering the matrix layer by means of the backing liner.

The coating mass preferably comprises the onion extract (A*) and the acrylate based polymer (B) or the thermoplastic hot melt adhesive composition (B*).

The acrylate based polymer is preferably provided in a solution or dispersion comprising at least one solvent $S^{\#}$. Thus, the method preferably comprises

a) providing the release liner,

b) applying a coating mass comprising an onion extract (A*) and

    -- and a mixture comprising an acrylate based polymer (B) and a solvent $S^{\#}$, or

    -- a thermoplastic hot melt adhesive composition (B**)

to the release liner,

c) drying the release liner and the coating mass applied thereto such that the matrix layer is formed on the release layer, said matrix layer comprising an onion extract (A) and

    -- an acrylate based polymer (B), or

    -- a thermoplastic hot melt adhesive (B*).

The solvent $S^{\#}$ us preferably an organic solvent, preferably an organic solvent selected from the group consisting of DMSO, hexane, toluene, xylene, cyclohexane, methylalcohol, isopropanol, 2,4 pentadione, ethyl acetate, heptane and mixtures of two or more thereof, preferably ethyl acetate and/or heptane.

As described above, the thermoplastic hot melt adhesive composition (B**) is preferably mixed with at least one solvent $S^{hm}$.

Preferably, the coating mass further comprises the at least one emulsifier. More preferably, the coating mass further comprises allantoin. Further, preferably, the coating mass may comprise at least one additional solvent, such as a solvent selected from the group consisting of ethylacetate, DMSO, heptane, ethanol, water and mixtures of two or more thereof, in addition to $S^{\#}$ or $S^{hm}$.

Preferably, before depositing the coating mass on the release liner, the coating mass is homogenized. Suitable methods for homogenizing the mass are known to those skilled in the art.

Preferably, in a first step, the onion extract and part of the polymer (B) or the thermoplastic hot melt adhesive composition (B**) are homogenized to give a mixture M1.

The homogenization in case of (B**) is preferably carried out in the presence of $S^{hm}$, preferably in ethyl acetate and/or heptane, as described above. The homogenization in case of (B) is preferably carried out in the presence of at least one solvent, optionally in addition to $S^{\#}$, preferably in the presence of ethyl acetate and/or heptane.

Likewise, part of the polymer (B) or of the thermoplastic hot melt adhesive composition (B**) and the allantoin are homogenized to give mixture M2. The homogenization in case of (B**) is preferably carried out in the presence of $S^{hm}$, preferably in ethyl acetate and/or heptane, and additionally in the presence of DMSO. The homogenization in case of (B) is also preferably carried out in the presence of at least one solvent, optionally in addition to $S^{\#}$, preferably in the presence of DMSO.

Preferably, to both mixtures an emulsifier is added.

Subsequent to the homogenization steps, M1 and M2 are mixed together, and preferably homogenized again to give the coating mass, said coating mass preferably comprising the at least one solvent L which is added in the first step and/or in the second step as described above, wherein the solvent is selected from the group consisting of ethylacetate, DMSO, heptane, ethanol, water and mixtures of two or more thereof, preferably wherein the solvent is a mixture of DMSO and ethanol, and wherein said coating mass optionally comprises $S^{\#}$ or $S^{hm}$

According to an alternative preferred embodiment, allantoin is dissolved in DMSO. Subsequently, the polymer (B), optionally comprising $S^{\#}$, or the thermoplastic hot melt adhesive composition (B**) is mixed with a solvent, such as an organic solvent, such as an organic solvent selected from the group consisting of preferably an organic solvent selected from the group consisting of DMSO, hexane, toluene, xylene, cyclohexane, methylalcohol, isopropanol, 2,4 pentadione, ethyl acetate, heptane and mixtures o two or more thereof, more preferably ethyl acetate and/or heptane. Then, preferably, the other components are added stepwise and the mixture is homogenized to give the coating mass.

As mentioned above, the thermoplastic hot melt adhesive composition (B**) is preferably not melted in order to apply the mass onto the release liner but dispersed or dissolved in the solvent $S^{hm}$.

Preferably, the coating mass is evenly applied to the release liner by means of a coating knife. This facilitates the formation of a matrix layer comprising a constant thickness. Thus, the active agents may be evenly released to the patient.

The drying is preferably carried out at a temperature in the range of from 30 to 150 °C, wherein the temperature may be varied or held essentially constant. Preferably, the temperature is continuously or stepwise increased during the drying step. Preferably, the drying is carried out in four drying zones having different temperatures. For examples, the four zones may comprise temperatures in a range from 30 °C to 150 °, preferably in a range from 40 °C to 140 °C and more preferably in a range from 50 °C to 120 °C. The temperature in each zone may be varied or held essentially constant. Further, the temperature may preferably increase from the first zone to the fourth zone. The drying may preferably be carried out over 15 to 20 minutes. Preferably, in the drying step, substantially all of the solvent present in the coating mass and/or the onion extract is removed. "Substantially all" is denoted to mean preferably at least 98 % by weight, more preferably at least 99 % by weight, more preferably at least 99.9 % by weight, more preferably all of the solvent is removed.

Preferably, the drying is carried it in four temperature zones, wherein in the first zone the temperature is in the range of from 35 °C to 45 °C, in the second zone the temperature is in the range of from 45 °C to 55 °C, in the third zone the temperature is in the range of from 85 °C to 95 °C, and wherein in the fourth zone the temperature is in the range of from 115 °C to 125 °C. Such drying process process is e.g. carried out in an oven in which the temperature is increased stepwise to achieve these different temperature zones.

Preferably, the drying in step (c) is carried out in a continuous coating line having four drying zones. Preferably, in this coating line, the drying is carried out with a web speed in the range of from 1.3 m/min to 1.8 m/min.

Preferably, the backing liner, the matrix layer and the release liner form a laminate, wherein the laminate is die cut so as to form the patch. Thus, the form of the patch is constant. Further, the die cutting may be carried out so as to die cut a plurality of patches which improves the production efficiency.

The backing liner and the matrix layer are die cut in a first die cutting step and the release liner is die cut in a second die cutting step, wherein the second die cutting step is carried out such that the release liner comprises a greater surface area than a surface of the matrix layer facing the release liner. Thus, the release liner protrudes from the matrix layer and protects the same in a reliable manner.

For example, the matrix layer comprises side faces and the second die cutting step is carried out such that the release liner protrudes from the side faces in a direction being perpendicular with respect to a direction of the layer composition. Thus, the release liner protrudes from all side faces from the matrix layer.

The release liner is die cut in a third die cutting step so as to form a release means in the release liner. The provision of a release means helps a user of the patch to release the release liner from the matrix layer.

The method may further comprise a sealing step, wherein the patch is sealed in a foil. Accordingly, the patch is reliably protected during storage such that the patch is prevented from being adversely influenced.

The method may further comprise a foil die cutting step, wherein the foil is die cut so as to form a pouch containing the patch. Thus, the patch may be packed in a single step.

Patch for use in a method of preventing or treating scars

It is to be understood that the preparation of the patch preferably takes place under GMP standardized conditions in order to ensure quality, pharmaceutical security, and effectiveness of the medical device. Further criteria for an ingredient being pharmaceutically acceptable can be derived from approval regulations by a regulatory agency or other generally recognized pharmacopoeias.

The patch according to the present invention is preferably for use in treating and/or preventing a scar.

The term "scar" is understood by the skilled person. As used herein, the term, preferably, refers to an abnormal morphological structure which results from a wound, in particular from wounds such as cuts, lacerations, abrasions, gunshot wounds, traumatic skin injury, penetration wounds, acne, operation wounds and burns. A scar typically comprises fibrous tissue. Preferably, the term "scar" includes hypertrophic scars, atrophic scars, and keloid scars. Atrophic scars are flat and depressed below the surrounding skin as a valley or hole. Usually, they are caused when underlying structures which support the skin, e.g. fat tissue or muscle tissue are lost. Hypertrophic scars are elevated scars. Hypertrophic scars occur when the body overproduces collagen. Thereby, the scar is raised above the surrounding skin. Keloid scars are elevated scars that spread beyond the margins of the original wound and invade the surrounding normal skin in a way that is site specific, and often contain whorls of collagen arranged in an abnormal fashion. As laid out elsewhere herein, it is particularly contemplated to treat scars shortly after scar formation. Moreover, it is preferred to treat scars which have been present over a longer period at the time at which treatment is initiated.

Moreover, the term "scar", preferably, also includes stretch marks. Stretch marks (frequently also referred to as striae) are a form of scarring caused by the overstretching of skin. This stretching disrupts the normal production of collagen and a scar results. Preferably, stretch marks are caused by rapid growth or rapid weight gain, in particular as a consequence of pregnancy. The term "stretch marks" preferably, includes striae distensae, striae atro phicans, striae rubra (red stretch marks) and striae alba (white stretch marks).

The terms "treating a scar" or "treatment of a scars", preferably, refers to the patch being use for therapeutic treatment or the cosmetic treatment of a scar. The cosmetic treatment, preferably, comprises the administration of the composition of the present invention to a subject with a scar for improving the appearance of scar tissue and/or for reduction of scar tissue. The improvement of appearance of scar tissue, preferably, refers to reduced discoloration, decreased hyperpigmentation, softening of scar tissue, decreased erythma, or improved aesthetic appearance of the scar. The reduction of scar tissue, preferably, refers to reduced scar height and/or scar size.

Preferably, the terms "treating a scar" or "treatment of scars" comprise patches for use in softening of scar tissue thus improving softness of scar tissue, improved aesthetic appearance of the scar, and, more preferably, reduced discoloration of the scar. Preferably, the discoloration of a scar is reduced if the redness of the scar is reduced. By reducing the discoloration, the scar becomes less visible.

As set forth above, the terms "treating a scar" or "treatment of scars" also refer to the patch being for use in a therapeutic treatment of a scar, in particular include amelioration or prevention of pain (in particular scar pain) and amelioration or prevention of pruritus.

The term "subject" as used herein relates to animals, preferably mammals, and, more preferably, humans.

Preferably, the patch of the present invention is for application onto the scar, i.e. to the scar tissue, or to the stretch mark.

According to a further embodiment of the invention, the patch of the present invention is for application onto the wound during the wound healing process. According to another embodiment of the invention, the patch of the present invention is for application onto the skin after completion of the wound healing process. In particular, the patch of the present invention can be applied onto the skin shortly after scar formation, e.g. within one month after scar formation. Further, it is contemplated to treat older scars which have been present over a longer period at the time at which treatment is initiated. Thus, the scar, preferably, has been present for at least one month, more preferably, for at least six months, or, most preferably, for at least one year at the time at which treatment is initiated.

Preferably, the composition of the present invention is for application over night, i.e. for at least 6, preferably at least 8 h.

It is to be understood that the composition of the present invention is for application for a time effective to allow for the treatment or prevention of a scar.

The patch of the present invention can be also for use in preventing scars, i.e. for preventing scar formation. The term "preventing" as used herein, preferably, refers to applying the patch to a subject in order to prevent or, in particular, to reduce *de novo* formation of scars. Moreover, the prevention of scars preferably also comprises the amelioration or prevention of pain and/or pruritus during scar formation. In order to prevent scars, the patch is administered to a wound while the wound is still in the process of healing and, thus, during completion of the wound healing process.

According to further embodiments of the invention, the patch of the present invention further exhibits a bactericidal activity. The term "bactericidal activity" as used herein, preferably refers to an activity which is capable killing bacterial cells. The patch of the present invention may, if for use in administration to a subject, advantageously be for use for treating and preventing scars and for killing bacterial cells in the scar tissue.

Also, the patch of the present invention may further for use in preventing excessive fibroblast proliferation.

Moreover, the patch of the present invention, preferably, further exhibits anti-inflammatory activity.

The present invention also relates to a patch according to the present invention for use for treating and/or preventing a scar in a subject in need thereof wherein the use comprises applying to the skin of said subject the patch of the present invention.

The present invention also relates to the patch of the present invention for treating and/or preventing a scar in a subject in need thereof.

The present invention also relates to a method for the cosmetic treatment of a scar in a subject in need thereof comprising applying to the skin of said subject the patch of the present invention.

The present invention also relates to the use of the patch of the present invention for the cosmetic treatment of a scar in a subject in need thereof.

As set forth above, cosmetic treatment, preferably, comprises the administration of the patch of the present invention to a subject with a scar for improving the appearance of scar tissue and/or for reduction of scar tissue. In particular, the term comprises softening of scar tissue, thus improving softness of scar tissue, improved aesthetic appearance of the scar, and reduced discoloration of the scar.

In the following, embodiments according to the present invention are described:

1. Patch comprising a layer composition, wherein the layer composition comprises at least

- a backing liner,
- a matrix layer comprising an onion extract (A), and

  - an acrylate based polymer (B), or
  - a thermoplastic hot melt adhesive (B*),

- and a release liner, wherein

the matrix layer is disposed between the backing liner and the release liner such that the matrix layer is contacted by the backing liner and the release liner on opposite sides thereof, wherein the release liner is releasable from the matrix layer.

2. Patch according to embodiment 1, wherein the matrix layer comprises the onion extract (A) in an amount in the range of from 0.01 to 5 % by weight, based on the total weight of the matrix layer and calculated as the dry onion extract.

3. Patch according to embodiment 1 or 2, wherein the onion extract (A) is an *Allium cepa* extract.

4. Patch according to any one of embodiments 1 to 3, wherein the matrix layer comprises an acrylate based polymer (B) and an onion extract (A), and wherein the polymer (B) is a acrylate-vinylacetate based polymer comprising building blocks derived from the monomeric components acrylate (B1) and vinylacetate (B2).

5. Patch according to any one of embodiments 1 to 3, wherein the matrix layer comprises a thermoplastic hot melt adhesive (B*), and an onion extract (A), and wherein the polymer (B*) is a hot melt thermoplastic rubber, preferably comprising a styrene polymer, a tackifying resin and a plasticizer.

6. Patch according to any one of embodiments 1 to 3 and 5, wherein the matrix layer comprises a thermoplastic hot melt adhesive (B*), and an onion extract (A), wherein the matrix layer is obtained, by

- dissolving or dispersing a thermoplastic hot melt adhesive composition (B**) in a solvent $S^{hm}$,
- mixing this solution or dispersion with at least an onion extract (A*) to give a coating mass,
- applying the coating mass onto a release liner, and
- drying the release liner and the coating mass applied thereto such that the matrix layer comprising an onion extract (A) and the thermoplastic hot melt adhesive (B*) is formed on the release layer, and comprising an onion extract (A*) and an thermoplastic hot melt adhesive (B*) to the release liner.

7. Patch according to embodiment 6, wherein the thermoplastic hot melt adhesive composition B** is not melted prior to the application of the coating mass onto the release liner.

8. Patch according to any one of embodiments 1 to 7, wherein the matrix layer further comprises at least one emulsifier, preferably caprylocaproyl macrogol glyceride.

9. Patch according to embodiment 8, wherein the matrix layer comprises the at least one emulsifier in an amount in the range of from 0.01 to 5 % by weight, based on the total weight of the matrix layer.

10. Patch according to any one of embodiments 1 to 9, wherein the matrix layer further comprises allantoin, preferably in an amount in the range of from 0.01 to 5 % by weight, based on the total weight of the matrix layer.

11. Patch according to any one of embodiments 1 to 10, wherein the release liner comprises a greater surface area than a surface of the matrix layer facing the release liner.

12. Patch according to any one of embodiments 1 to 11, wherein the release liner protrudes from the matrix layer in at least one direction being perpendicular with respect to a direction of the layer composition.

13. Patch according to any one of embodiments 1 to 12, wherein the matrix layer comprises side faces and the release liner protrudes from the side faces in a direction being perpendicular with respect to a direction of the layer composition.

14. Patch according to any one of embodiments 1 to 13, wherein the matrix layer is disposed substantially symmetrically on the release liner.

15. Patch according to any one of embodiments 1 to 14, wherein the backing liner fully covers a surface of the matrix facing the baking liner.

16. Patch according to any to any one of embodiments 1 to 15, wherein the backing liner is at least partially made of an occlusive material.

17. Patch according to any one of embodiments 1 to 16, wherein the backing liner is a foam liner, preferably a foam liner comprising a polyolefin.

18. Patch according to any one of embodiments 1 to 17, wherein the backing liner comprises a greater thickness than the matrix layer.

19. Patch according to any one of embodiments 1 to 18, wherein the backing liner comprises a thickness in a range of from 150 $\mu$m to 500 $\mu$m and preferably in a range of from 200 $\mu$m to 400 $\mu$m, more preferably in the range of from 200 $\mu$m to 400 $\mu$m and still more preferably 300 $\mu$m.

20. Patch according to any one of embodiments 1 to 19, wherein the release liner is at least partially siliconized.

21. Patch according to any one of embodiments 1 to 20, wherein the release liner comprises a thickness in a range of from 25 $\mu$m to 75 $\mu$m.

22. Patch according to any one of embodiments 1 to 21, wherein the backing liner and/or the matrix layer and/or the release liner comprises a rectangular surface if seen in a direction of the layer composition.

23. Patch according to any one of embodiments 1 to 22, wherein the backing liner is adhered to the matrix layer.

24. Patch according to any one of embodiments 1 to 23, wherein the backing liner is permanently adhered to the matrix layer.

25. Method for manufacturing a patch comprising the following steps:

> (a) providing the release liner,
> (b) applying a coating mass comprising an onion extract (A*) and
>
> > -- an acrylate based polymer (B), or
> > -- a thermoplastic hot melt adhesive composition (B**)
>
> to the release liner,
> (c) drying the release liner and the coating mass applied thereto such that the matrix layer is formed on the release layer, said matrix layer comprising an onion extract (A) and
>
> > -- an acrylate based polymer (B), or
> > -- a thermoplastic hot melt adhesive (B*)
>
> (d) and covering the matrix layer by means of the backing liner.

26. Method according to embodiment 25, wherein the onion extract (A*) of step (b) is obtainable by a process comprising the steps

> (a1) extracting fresh or dried onions, preferably *Allium cepa,* with a solvent $S_1$ comprising at least one alcohol to give a liquid phase $L_1$ and a solid residue $R_o$,
> (b1) separating the liquid phase $L_1$ from the solid residue $R_o$,
> (c1) evaporating the liquid phase $L_1$ to give a residue $R_1$,
> (d1) redissolving the residue $R_1$ in a solvent $S_2$ comprising at least one alcohol and water to give the onion extract (A*)

27. Method according to embodiment 26, wherein the coating mass comprises a thermoplastic hot melt adhesive, and wherein the step (a) comprises

> - dissolving or dispersing the thermoplastic hot melt adhesive composition (B**) in a solvent $S^{hm}$, mixing this solution or dispersion with at least an onion extract (A*)

to give the coating mass, and applying said coating mass to the release liner.

28. Method according to any one of embodiments 22 to 27, wherein the coating mass of step (b) further comprises at least one emulsifier.

29. Method according to any one of embodiments 25 to 28, wherein the coating mass of step (b) further comprises allantoin.

30. Method according to any one of embodiments 25 to 29, wherein in step (b) the coating mass is evenly applied to the release liner by means of a coating knife.

31. Method according to any one of embodiments 25 to 30, wherein the drying in step (c) is carried out in four temperature zones having different temperatures.

32. The method according to embodiment 31, wherein in the first zone the temperature is in the range of from 35 °C to 45 °C, in the second zone the temperature is in the range of from 45 °C to 55 °C, in the third zone the temperature is in the range of from 85 °C to 95 °C, and wherein in the fourth zone the temperature is in the range of from 115 °C to 125 °C.

33. The method according to embodiment 31 or 32, wherein the drying in step (c) is carried out in a continuous coating line having four drying zones and the drying is performed with a web speed in the range of from 1.3 m/min to 1.8 m/min.

34. Method according to any one of embodiments 25 to 33, wherein the backing liner, the matrix layer and the release liner form a laminate, wherein the laminate is die cut so as to form the patch.

35. Method according to embodiment 34, wherein the backing liner and the matrix layer are die cut in a first die cutting step and the release liner is die cut in a second die cutting step, wherein the second die cutting step is carried out such that the release liner comprises a greater surface area than a surface of the matrix layer facing the release liner.

36. Method according to embodiment 34 or 35, wherein the matrix layer comprises side faces and the second die cutting step is carried out such that the release liner protrudes from the side faces in a direction being perpendicular with respect to a direction of the layer composition.

37. Method according to any one of embodiments 35 to 36, wherein the release liner is die cut in a third die cutting step so as to form a release means in the release liner.

38. Method according to any one of embodiments 25 to 37, further comprising a sealing step, wherein the patch is sealed in a foil.

39. Method according to any one of embodiments 25 to 38, further comprising a foil die cutting step, wherein the foil is die cut so as to form a pouch containing the patch.

40. Patch obtained or obtainable by a method according to any one of embodiments 25 to 39.

41. Patch according to any one of embodiments 1 to 24 or embodiment 40 for use in treating and/or preventing a scar.

**Figures:**

Further details and aspects of the present invention may be realized from the below description of preferred embodiments which are schematically shown in the drawings, in which:

Fig. 1 shows a cross-sectional view of a patch according to an embodiment of the present invention;

Fig. 2 shows a top view of the patch;

Fig. 3 shows a first manufacturing step for manufacturing a patch according to an embodiment of the present invention;

Fig. 4 shows a second manufacturing step for manufacturing a patch according to an embodiment of the present invention;

Fig. 5 shows a third manufacturing step for manufacturing a patch according to an embodiment of the present invention;

Fig. 6 shows a fourth manufacturing step for manufacturing a patch according to an embodiment of the present invention;

Fig. 7 shows a fifth manufacturing step for manufacturing a patch according to an embodiment of the present invention;

Fig. 8 shows a sixth manufacturing step for manufacturing a patch according to an embodiment of the present invention; and

Fig. 9 shows a seventh manufacturing step for manufacturing a patch according to an embodiment of the present invention.

**Examples:** Detailed description of the patch

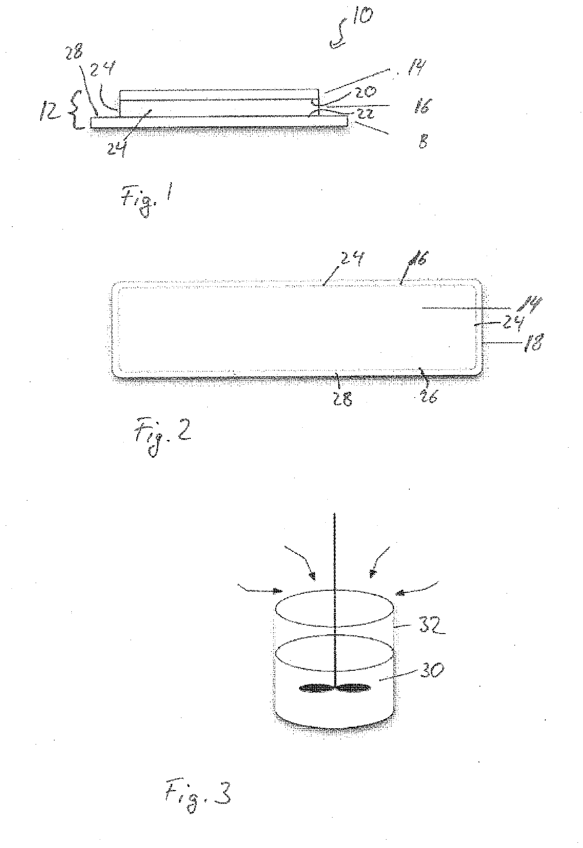

Fig. 1 shows a schematic cross-section of a patch 10 according to an embodiment of the present invention. The patch 10 may be used for the treatment of scars. The patch 10 comprises a layer composition 12. The layer composition 12 comprises a backing liner 14, a matrix layer 16 and a release liner 18.

The backing liner 14 may be specified as described in example 1

The matrix layer 16 may be specified as described in example 1

The release liner 18 may be specified as described in example 1

As shown in Fig. 1, the matrix layer 16 is disposed between the backing liner 14 and the release liner 18 such that the matrix layer 16 is contacted by the backing liner 14 and the release liner 18 on opposite sides thereof. Thus, the matrix layer 16 is sandwiched between the backing liner 14 and the release liner 18. The release liner 18 is releasable from the matrix layer 16. Each of the backing liner 14, the matrix layer 16 and the release liner 18 is formed as a thin cuboid. Thus, the contacting surfaces of the backing liner 14, the matrix laxer 16 and the release liner 18 are rectangular. The edges of the rectangular shape may be rounded. Particularly, the matrix layer 16 comprises a top side 20 contacting the backing liner 14, a bottom side 22 contacting the release liner 18 and side faces 24 being perpendicular to the top side 20 and the bottom side 22.

The backing liner 14 fully covers a surface of the matrix layer 16 facing the backing liner 14. In this embodiment, the surface of the matrix layer 16 facing the backing liner 14 is the top side 20 as mentioned above. The backing liner 14 may comprise a length of 12 cm and a width of 3 cm. Thus, the contacting surface area is 36 cm$^2$. Further, the backing liner 14 may comprise a thickness in a range from 150 $\mu$m to 500 $\mu$m and preferably in a range from 200 $\mu$m to 400 $\mu$m, more preferably in the range from 200 $\mu$m to 400 $\mu$m. For example, the backing liner comprises a thickness of 300 $\mu$m. The backing liner 14 comprises a greater thickness than the matrix layer 16 as will be described in more detail below. The backing liner 14 is at least partially made of an occlusive material. The backing liner 14 is adhered to the matrix layer 16. Preferably, the backing liner 14 is permanently adhered to the matrix layer 16.

The release liner 18 comprises a greater surface area than a surface of the matrix layer 16 facing the release liner 18. In this embodiment, the surface of the matrix layer 16 facing the release liner 18 is the bottom side as mentioned above. Fig. 2 shows a top view of the patch 10. As shown in Fig. 2, the release liner 18 protrudes from the matrix layer 16 in at least one direction being perpendicular with respect to a direction of the layer composition 12. Particularly, the release liner 18 protrudes from the side faces 24 of the matrix layer 16 in a direction being perpendicular with respect to a direction of the layer composition 12. Thus, the matrix layer 16 is disposed substantially symmetrically on the release liner 18. With other words, the matrix layer 16 is evenly spaced apart from edges 26 of the release liner 18. For example, the release liner 18 comprises a length of 14 cm and a width of 4 cm. Thus, the release liner 18 evenly protrudes from the side faces 24 of the matrix layer 16 at 5 mm. The release liner 18 comprises a thickness in a range from 25 $\mu$m to 75 $\mu$m. For example, the release liner 18 comprises a thickness of 50 $\mu$m. The release liner 18 is at least partially siliconized. For example, a top side 28 of the release liner 18, onto which the matrix laxer 16 is disposed, is siliconized. This ensures that the release liner 18 may be easily released from the matrix layer 16 for using the patch 10. The release liner 18 may further comprise a release means die cut therein which facilitates releasing of the release liner 18. For example, the release liner 18 may comprise a latch or a perforation.

The patch 10 may be manufactured as will be explained in more detail below. As shown in Fig. 3, which shows a first step of the manufacturing process, a coating mass 30 may be provided. For example, the coating mass may be provided by stirring the above ingredients in a container 32 such as a vessel.

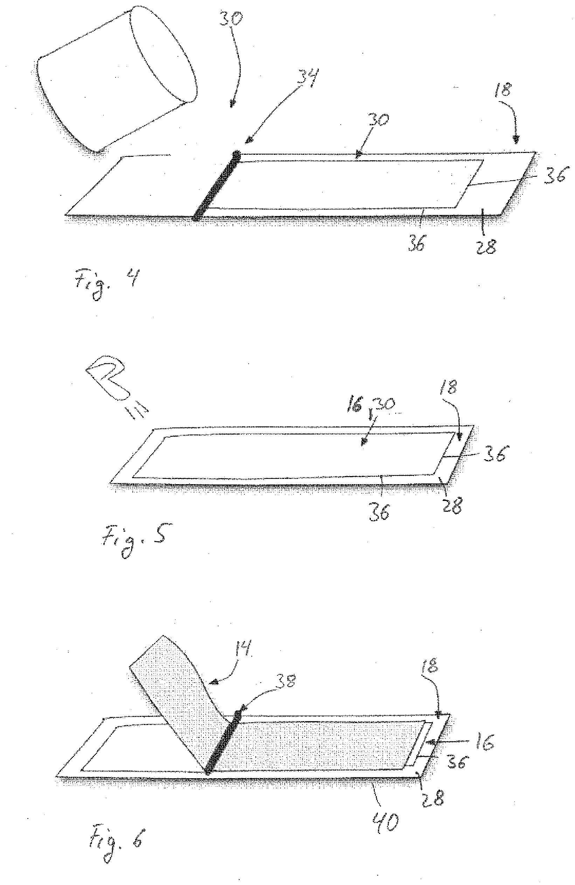

Fig. 4 shows a second step of the manufacturing process. A release liner 18 as explained above is provided. The coating mass 30 is applied to the release liner 18. More particularly, the coating mass 30 is applied to the siliconized top side 28 of the release liner 18. Further, the coating mass 30 is evenly applied to the release liner 18 by means of a coating knife 34. Alternatively, a nozzle may be used for applying the coating mass 30. The coating mass 30 is applied to the release liner 18 so as to have a thickness in a range from 20 $\mu$m to 100 $\mu$m and preferably in a range from 25 $\mu$m to 80 $\mu$m, more preferably in the range from 30 $\mu$m to 60 um, and more preferably of 40 $\mu$m.

As shown in Fig. 5, which shows a third step of the manufacturing process, the coating mass 30 may be applied to the release liner 18 such that the release liner 18 protrudes from edges 36 of the coating mass 30. With other words, the release liner 18 comprises a greater surface area than a surface area of the coating mass 30 contacting the release liner 18. Further, the release liner 18 and the coating mass 30 applied thereto are dried such that solvent is released from the coating mass 30 and the above described matrix layer 16 is formed on the release liner 18. The drying may be carried out in a drying tunnel having four drying zones. The temperature in the four drying zones may be in a preferred range of from 50 °C to 120 °C over 15 to 20 minutes. The temperature may increase from the first zone to the fourth zone which corresponds to an increase in the conveying direction of the release liner 18 and the coating mass 30. The backing liner 14 and/or the matrix layer 16 may be provided with an adhesive such that the backing liner 14 is permanently adhered to the matrix layer 16.

Fig. 6 shows a fourth step of the manufacturing process. As shown in Fig. 6, the above described backing liner 14 is applied to the matrix layer 16 so as to cover the same. More particularly, the backing liner 14 may be applied to the matrix layer 16 by means of a lamination roll 38 which facilitates an even pressure distribution onto the backing liner 14 and the matrix layer 16. Thus, the backing liner 14, the matrix layer 16 and the release liner 18 form a laminate 40 which may be considered as a large patch band. Subsequently, the laminate 40 may be reeled up and transported to a die cutting station. Needless to say, the laminate 40 may be die cut immediately following the fourth step.

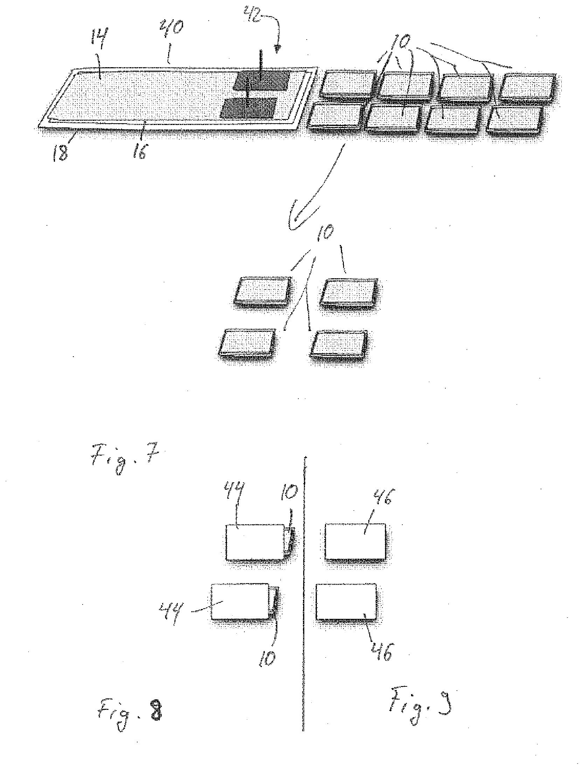

Fig. 7 shows a fifth step of the manufacturing process. As shown in Fig. 7, the laminate 40 is die cut by means of a die cutting tool 42. More particularly, the backing liner 14 and the matrix layer 16 are die cut in a first die cutting step such that the backing liner 14 and the matrix layer 16 comprise the above surface area. Then, the release liner 18 is die cut in a second die cutting step such that the release liner comprises the above surface area being greater than the surface area of the backing liner 14 and the matrix layer 16 facing the release liner 18. With other words, the release liner 18 evenly protrudes from the side faces 24 of the matrix layer 16 as explained above. It is explicitly mentioned that the second die cutting step may be made before the first die cutting step. Thus, from the laminate, a plurality of patches 10 is die cut having the above described layer composition 12. Further, the release liner 18 of each patch 10 protrudes from the side faces 24 of the matrix layer in a direction perpendicular with respect to a direction of the layer composition 12.

Furthermore, the release liner 18 may be die cut in a third die cutting step so as to form a release means as described above.

Fig. 8 shows a sixth step of the manufacturing process. As shown in Fig. 8, the process may further comprise a sealing step. In the sealing step, the patch 10 is sealed in a foil 44. Thus, the foil completely houses the patch 10 in a hermetical manner.

Fig. 9 shows a seventh step of the manufacturing process. As shown in Fig. 9, the process may further comprise a foil die cutting step. In the foil die cutting step, the foil 44 containing the patch 10 is die cut so as to form a pouch 46 containing the patch 10. Thus, The patch 10 is packed and the pouch 46 may be opened for taking out the patch 10 for use.

The laminate 40 may be cut before the die cutting steps to provide a plurality of laminates. The width of the cut laminate 40 may be adapted to the width of the width of the patch to be die cut.

**Example 1: Preparation of an acrylate based patch comprising onion extract:**

0.4 g allantoin were predissolved in DMSO (10 %) and were added to 43.96 g Duro Tak 87-2194 (solid content 43 %). A second preparation contained 1.96 g onion extract (30 % by weight in aqueous ethanolic solution (10-16 Vol-% ethanol in water), 1.2 g Labrasol and 43.96 g Duro Tak 87-2194. Both mixtures were merged together and adjusted with ethyl acetate to a solid content of 42.6 %. The mixture was stirred until homogenous by means of a mechanical stirrer. This coating mass was deposited in form of a thin layer by means of a suitable coating device (e.g. doctor blade) on an inert protective foil (PET film 50 $\mu$m, siliconized) and dried for 15-20 min at 50-120 °C. Subsequent to the drying step, the adhesive layer (area weight of 50 $g/m^2$) of the double layered laminate was laminated with a second foil (backing layer, e.g. polyolefine).

From the three layered laminate, patches of respective size (in this case 36 $cm^2$) were die cut by means of a rotating punch.

The onion extract is obtainable for example from Finzelberg Extrakte and described in US 5,885,581.

A patch comprising the following composition was obtained:

| | | Weight % based on the total weight of the respective layer | Per m$^2$ of the patch [g] |
|---|---|---|---|
| Matrix layer: Thickness (40 $\mu$m) | Onion extract | 1 | 0,4 |
| | Polyacrylatevinylacetate (Duro-Tak 87-2194) | 95 | 38 |
| | Labrasol | 3 | 1,2 |
| | Allantoin | 1 | 0,4 |
| Backing layer Thickness (500 $\mu$m) | Foam: Polyethylene foam: Alveolit TEE Z 0800.52 | | 65 |
| Release layer Thickness (50 $\mu$m) | PET film 50 $\mu$m, siliconized | | 72 |

The patch had an adhesive force of around 0.8 N/cm, measured by FMT-2 with a release rate of allantoin of 19 % and a release of onion extract of 28 %, based on the total weight of each compound in the patch, respectively, and measured after 6 h using HPLC.

Further, the patch was comfortable to wear and easy to remove from the skin while remaining secure in position. Further, biocompatibility according to ISO 10993-5 2009 is given.

**Comparative Example 2: Preparation of a silicone based patch comprising onion extract:**

| | | Weight % based on the total weight of the respective layer | Per m$^2$ of the patch [g] |
|---|---|---|---|
| Matrix layer: Thickness (40 $\mu$m) | Onion extract | 1 | 0,4 |
| | Silicon soft skin adhesive MG7-9900 Part A + Part B | Part A 49<br><br>Part B 49 | 39,2 |
| | Allantoin | 1 | 0,4 |
| Backing layer Thickness (500 $\mu$m) | Foam: Polyethylene foam: Alveolit TEE Z 0800.52 | | 65 |
| Release layer Thickness: (50 $\mu$m) | PET film 50 $\mu$m, siliconized | | 72 |

With silicone patches, no release of the onion extract was observed.

**Example 3: Preparation of a hot melt adhesive based patch comprising onion extract:**

Dermatak H542B (Henkel) has been dissolved in an ethylacetat/heptane mixture 1:1 (weight ratio of Dermatak to solvent mixture 1:1).

0.4 g Allantoin were predissolved in DMSO (10 %) and were added to 38,4 g Dermatak (solid content 50 %) ethyl acetate/heptane mixture. A second preparation contained 1.96 g onion extract (30 % by weight in aqueous ethanolic solution (10-16 Vol-% ethanol in water)), 0.8 g Labrasol and 38,4 g Dermatak ethyl acetate/heptane mixture (solid content 50 %). Both mixtures were merged together and adjusted with ethyl acetate to a solid content of 48 %. The mixture was stirred until homogenous by means of a mechanical stirrer. This coating mass was deposited in form of a thin layer by means of a suitable coating device (e.g. doctor blade) on an inert protective foil (PET film 50 $\mu$m, siliconized) and dried for 15-20 min at 50-120 °C. Subsequent to the drying step, the adhesive layer (area weight of 50 g/m$^2$) of the double layered laminate was laminated with a second foil (backing layer, e.g. polyolefine).

From the three layered laminate, patches of respective size (in this case 36 cm$^2$) were die cut by means of a rotating punch.

A patch comprising the following composition was obtained:

| | | Weight % based on the total weight of the respective layer | Per m$^2$ of the patch [g] |
|---|---|---|---|
| Matrix layer: Thickness (40 μm) | Onion extract | 1 | 0,4 |
| | Dermatak | 96 | 38,4 |
| | Labrasol | 2 | 0,8 |
| | Allantoin | 1 | 0,4 |

| Backing layer Thickness (500 μm) | Foam: Polyethylene foam: Alveolit TEE Z 0800.52 | 65 |
|---|---|---|
| Release layer Thickness (50 μm) | PET film 50 μm, siliconized | 72 |

### Example 4: Influence of drying temperature on onion extract content (cepalin content):

Dermatak H542B (Henkel) has been dissolved in an ethylacetat/heptane mixture 1:1 (weight ratio of Dermatak to solvent mixture 1:1).

0.44 Allantoin were predissolved in DMSO (10 %). The Allantoin mixture and 1.96 g onion extract (30 % by weight in aqueous ethanolic solution (10-16 Vol-% ethanol in water)), were added to 70.76 Dermatak ethyl acetate/heptane mixture (solid content 50 %). The mixture was adjusted with ethyl acetate to a solid content of 49 % stirred until homogenous by means of a mechanical stirrer. This coating mass was deposited in form of a thin layer by means of a suitable coating device (e.g. doctor blade) on an inert protective foil (PET film 50 μm, siliconized) and dried in dried for 15-20 min at 50-120 °C. Subsequent to the drying step, the adhesive layer (area weight of 50 g/m$^2$) of the double layered laminate was laminated with a second foil (backing layer, e.g. polyolefine).

From the three layered laminate, patches of respective size (in this case 36 cm$^2$) were die cut by means of a rotating punch.

In all obtained patches, the DMSO content and the cepalin content (content of the onion extract was measured). An optimum combination of a relative high amount of cepalin with a relative low amount of DMSO was obtained when using the temperatures and web speed shown in entry 4 and 5. At very high temperatures, the amount of onion extract was lower (entries 1 to 3), and at lower temperatures, the DMSO content higher (entry 6). Thus, a medium temperature together with a medium or low web speed is particularly advantageous.

| Entry | Temperature (°C) zone 1 - zone 2 - zone 3 - zone 4 | Web speed [m/min] | DMSO content (weight %, based on the total weight of the matrix layer) | Cepalin content (weight %, based on the total weight of the matrix layer) |
|---|---|---|---|---|
| 1 | 50 - 90 - 120 - 140 | 1.3 | 0 | 0.6 |
| 2 | 50 - 90 - 120 - 140 | 1.8 | 0 | 0.6 |
| 3 | 50 - 90 - 120 - 140 | 2.1 | 0 | 0.6 |
| 4 | 40 - 50 - 90 - 120 | 1,3 | 0.8 | 1.1 |
| 5 | 40 - 50 - 90 - 120 | 1,8 | 1.7 | 1.2 |
| 3 | 40 - 50 - 65 - 80 | 1,3 | 9.3 | 1.4 |

### Example 5: Further prepared patches

All samples had a release liner consisting of PET 50 micrometer siliconised (Primeliner, Loparex). The samples vary in the adhesive system and the backing layer material, but have all an Allantoin and Onion extract content of 1 %. All versions are summarized in the following table. Each single patch was packed in a peel-able pouch made of a PET-Alu-PP 3-composite layered foil.

The samples were manufactured with the parameter setting described above in example 1 and 3, respectively

| Batch | Sample | Adhesiv | Exipients | Backing |
|---|---|---|---|---|
| C0047 | 1 | DT 87-2677 | -- | TEE Z 0800.52 Foam |
| C0048 | 2 | DT 87-2194 | 3 % Labrasol | TEE Z 0800.52 Foam |
| C0057 | 3 | Dermatak H524B | -- | TEE Z 0800.52 Foam |
| C0050 | 4 | Dermatak H524B | 2 % Solutol | TEE Z 0800.52 Foam |
| C0051 | 5 | Dermatak H524B | 2 % Labrasol | TEE Z 0800.52 Foam |
| C0058 | 6 | Dermatak H524B | 4 % Peceol | Cotran 9720 |
| C0053 | 7 | DT 87-2677 | -- | Cotran 9720 |
| C0054 | 8 | DT 87-2194 | 3 % Labrasol | Cotran 9720 |
| C0055 | 9 | Dermatak H524B | -- | Cotran 9720 |
| C0056 | 10 | Dermatak H524B | 2 % Solutol | Cotran 9720 |

*C0048 corresponds to example 1, C0051 corresponds to example 3.

**Example 6: Cepalin and Allantoin release of the patches according to example 5**

The cepalin and allantoin release of the patches prepared according to example 6 was analyzed. The release study was carried out in 120 mL phosphate buffer pH 3 comprising 5% DMSO. The patch was incubated (thereby agitated) for 6h in the phosphate buffer. Thereafter, the amount of allantoin and cepalin present in the phosphate buffer was measured using HPLC .

Further, the adhesion force of the patches and DMSO content of the matrix layer was determined. The adhesion force was measured using the 90° peel adhesion test (FINAT test method no. 2 (FTM 2)) and the DMSO content using HPLC.

The results are given in the following table:

| Batch | Sample | Content Allantoin (%) | | Release (%) | | Adhesion force (N/cm) | DMSO content (%) |
|---|---|---|---|---|---|---|---|
| | | Allantoin | Cepalin | Allantoin | Cepalin | | |
| C0047 | 1 | 0.91 | 0.49 | 14.5 | 94.0 | - | - |
| C0048 | 2 | 0.93 | 1.22 | 18.9 | 27.6 | 0.77 | 0.09 |
| C0057 | 3 | 0.90 | 0.85 | 3.8 | 10.8 | - | - |
| C0050 | 4 | 1.02 | 0.85 | 50.6 | 24.7 | 2.21 | 0.37 |
| C0051 | 5 | 0.83 | 0.85 | 4.2 | 15.2 | - | - |
| C0058 | 6 | 1.17 | 1.10 | 20.4 | 24.0 | 2.03 | 0.74 |
| C0053 | 7 | 1.07 | 0.49 | 17.2 | 86.4 | - | - |
| C0054 | 8 | 1.04 | 1.22 | 32.6 | 14.8 | 0.73 | 0.09 |
| C0055 | 9 | 0.98 | 0.85 | 15.4 | 17.1 | 2.86 | 0.39 |
| C0056 | 10 | 0.96 | 0.98 | 28.5 | 26.2 | 2.95 | 0.28 |

All patches comprised onion extract and allantoin and were able to release up to 94 % of the total amount of the onion extract as well as of cepalin.

In particular, samples 2, 4, 6, 8, 9 and 10 showed an advantageous combination of a high amount of allantoin and cepalin within the patches, with a relatively low amount of DMSO and with a relatively high release of cepalin and allantoin when used.

**Claims**

1. Patch comprising a layer composition, wherein the layer composition comprises at least

   - a backing liner,
   - a matrix layer comprising an onion extract (A), and

      - an acrylate based polymer (B), or
      - a thermoplastic hot melt adhesive (B*),

   - and a release liner, wherein

   the matrix layer is disposed between the backing liner and the release liner such that the matrix layer is contacted by the backing liner and the release liner on opposite sides thereof, wherein the release liner is releasable from the matrix layer.

2. Patch according to claim 1, wherein the matrix layer comprises the onion extract (A) in an amount in the range of from 0.01 to 5 % by weight, based on the total weight of the matrix layer and calculated as the dry onion extract.

3. Patch according to claims 1 or 2, wherein the onion extract (A) is an *Allium cepa* extract.

4. Patch according to any one of claims 1 to 3, wherein the matrix layer comprises an acrylate based polymer (B) and an onion extract (A), and wherein the polymer (B) is a acrylate-vinylacetate based polymer comprising building blocks derived from the monomeric components acrylate (B1) and vinylacetate (B2).

5. Patch according to any one of claims 1 to 3, wherein the matrix layer comprises a thermoplastic hot melt adhesive (B*), and an onion extract (A), and wherein the polymer (B*) is a hot melt thermoplastic rubber, preferably comprising a styrene polymer, a tackifying resin and a plasticizer.

6. Patch according to any one of claims 1 to 3 and 5, wherein the matrix layer comprises a thermoplastic hot melt adhesive (B*), and an onion extract (A), wherein the matrix layer is obtained, by

   - dissolving or dispersing a thermoplastic hot melt adhesive composition (B**) in a solvent $S^{hm}$,
   - mixing this solution or dispersion with at least an onion extract (A*) to give a coating mass,
   - applying the coating mass onto a release liner, and
   - drying the release liner and the coating mass applied thereto such that the matrix layer comprising an onion extract (A) and the thermoplastic hot melt adhesive (B*) is formed on the release layer, and comprising an onion extract (A*) and an thermoplastic hot melt adhesive (B*) to the release liner.

7. Patch according to claim 6, wherein the thermoplastic hot melt adhesive composition B** is not melted prior to the application of the coating mass onto the release liner.

8. Patch according to any one of claims 1 to 7, wherein the matrix layer further comprises allantoin, preferably in an amount in the range of from 0.01 to 5 % by weight, based on the total weight of the matrix layer.

9. Patch according to any to any one of claims 1 to 8, wherein the backing liner is at least partially made of an occlusive material, and/or wherein the backing liner is a foam liner, preferably a foam liner comprising a polyolefin.

10. Method for manufacturing a patch comprising the following steps:

    (a) providing the release liner,
    (b) applying a coating mass comprising an onion extract (A*) and

       -- an acrylate based polymer (B), or
       -- a thermoplastic hot melt adhesive composition (B**)

    to the release liner,
    (c) drying the release liner and the coating mass applied thereto such that the matrix layer is formed on the release layer, said matrix layer comprising an onion extract (A) and

-- an acrylate based polymer (B), or
-- a thermoplastic hot melt adhesive (B*)

(d) and covering the matrix layer by means of the backing liner.

11. Method according to claim 10, wherein the onion extract (A*) of step (b) is obtainable by a process comprising the steps

(a1) extracting fresh or dried onions, preferably *Allium cepa,* with a solvent $S_1$ comprising at least one alcohol to give a liquid phase $L_1$ and a solid residue $R_0$,
(b1) separating the liquid phase $L_1$ from the solid residue $R_0$,
(c1) evaporating the liquid phase $L_1$ to give a residue $R_1$,
(d1) redissolving the residue $R_1$ in a solvent $S_2$ comprising at least one alcohol and water to give the onion extract (A*)

12. Method according to claim 10 or 11, wherein the coating mass comprises a thermoplastic hot melt adhesive, and wherein the step (a) comprises

- dissolving or dispersing the thermoplastic hot melt adhesive composition (B**) in a solvent $S^{hm}$, mixing this solution or dispersion with at least an onion extract (A*) to give the coating mass, and applying said coating mass to the release liner.

13. Method according to any one of claims 10 to 12, wherein the drying in step (c) is carried out in four drying zones having different temperatures, preferably wherein in the first zone the temperature is in the range of from 35 °C to 45 °C, in the second zone the temperature is in the range of from 45 °C to 55 °C, in the third zone the temperature is in the range of from 85 °C to 95 °C, and wherein in the fourth zone the temperature is in the range of from 115 °C to 125 °C.

14. The method according to claim 12 or 13, wherein the drying in step (c) is carried out in a continuous coating line having four temperature zones and the drying is performed with a web speed in the range of from 1.3 m/min to 1.8 m/min.

15. Patch according to any one of claims 1 to 9 obtained or obtainable by a method according to any one of claims 10 to 14.

16. Patch according to any one of claims 1 to 9 or claim 15 for use in treating and/or preventing a scar.


**Patentansprüche**

1. Pflaster, umfassend eine Schichtzusammensetzung, wobei die Schichtzusammensetzung mindestens umfasst:

- eine rückseitige Auflage,
- eine Matrixschicht, umfassend einen Zwiebelextrakt (A) und

- ein auf Acrylat basierendes Polymer (B) oder
- einen thermoplastischen heißschmelzenden Klebstoff (B*),

- und eine Abziehauflage, wobei

die Matrixschicht zwischen der rückseitigen Auflage und der Abziehauflage derart angeordnet ist, dass die Matrixschicht von der rückseitigen Auflage und der Abziehauflage auf gegenüberliegenden Seiten davon kontaktiert wird, wobei die Abziehauflage von der Matrixschicht abgezogen werden kann.

2. Pflaster nach Anspruch 1, wobei die Matrixschicht den Zwiebelextrakt (A) in einer Menge im Bereich von 0,01 bis 5 Gew.-%, bezogen auf das Gesamtgewicht der Matrixschicht und berechnet als trockener Zwiebelextrakt, umfasst.

**3.** Pflaster nach Anspruch 1 oder 2,
wobei der Zwiebelextrakt (A) ein *Allium cepa*-Extrakt ist.

**4.** Pflaster nach einem der Ansprüche 1 bis 3,
wobei die Matrixschicht ein auf Acrylat basierendes Polymer (B) und einen Zwiebelextrakt (A) umfasst, und wobei das Polymer (B) ein auf Acrylat-Vinylacetat basierendes Polymer ist, das Bausteine umfasst, die von den monomeren Komponenten Acrylat (B1) und Vinylacetat (B2) abgeleitet sind.

**5.** Pflaster nach einem der Ansprüche 1 bis 3,
wobei die Matrixschicht einen thermoplastischen heißschmelzenden Klebstoff (B*) und einen Zwiebelextrakt (A) umfasst, und wobei das Polymer (B*) ein heißschmelzender thermoplastischer Kautschuk ist, vorzugsweise umfassend ein Styrol-Polymer, ein klebrigmachendes Harz und einen Weichmacher.

**6.** Pflaster nach einem der Ansprüche 1 bis 3 und 5,
wobei die Matrixschicht einen thermoplastischen heißschmelzenden Klebstoff (B*) und einen Zwiebelextrakt (A) umfasst, wobei die Matrixschicht erhalten wird durch:

- Lösen oder Dispergieren einer thermoplastischen heißschmelzenden Klebstoffzusammensetzung (B**) in einem Lösungsmittel $S^{hm}$,
- Mischen dieser Lösung oder Dispersion mit mindestens einem Zwiebelextrakt (A*), um eine Beschichtungsmasse zu erhalten,
- Aufbringen der Beschichtungsmasse auf einer Abziehauflage, und
- Trocknen der Abziehauflage und der darauf aufgebrachten Beschichtungsmasse, so dass die Matrixschicht, umfassend einen Zwiebelextrakt (A) und den thermoplastischen heißschmelzenden Klebstoff (B*), auf der Abziehschicht gebildet wird, und umfassend einen Zwiebelextrakt (A*) und einen thermoplastischen heißschmelzenden Klebstoff (B*) auf der Abziehauflage.

**7.** Pflaster nach Anspruch 6,
wobei die thermoplastische heißschmelzende Klebstoffzusammensetzung B** vor dem Aufbringen der Beschichtungsmasse auf der Abziehauflage nicht geschmolzen ist.

**8.** Pflaster nach einem der Ansprüche 1 bis 7,
wobei die Matrixschicht ferner Allantoin, vorzugsweise in einer Menge im Bereich von 0,01 bis 5 Gew.-%, bezogen auf das Gesamtgewicht der Matrixschicht, umfasst.

**9.** Pflaster nach einem der Ansprüche 1 bis 8,
wobei die rückseitige Auflage mindestens teilweise aus einem okklusiven Material hergestellt ist, und/oder wobei die rückseitige Auflage eine Schaumauflage, vorzugsweise eine Schaumauflage, die ein Polyolefin umfasst, ist.

**10.** Verfahren zur Herstellung eines Pflasters, umfassend die folgenden Schritte:

(a) Bereitstellen der Abziehauflage,
(b) Aufbringen einer Beschichtungsmasse, umfassend einen Zwiebelextrakt (A*) und

-- ein auf Acrylat basierendes Polymer (B) oder
-- eine thermoplastische heißschmelzende Klebstoffzusammensetzung (B**),

auf der Abziehauflage,
(c) Trocknen der Abziehauflage und der darauf aufgebrachten Beschichtungsmasse, so dass die Matrixschicht auf der Abziehschicht gebildet wird, wobei die Matrixschicht einen Zwiebelextrakt (A) und

-- ein auf Acrylat basierendes Polymer (B) oder
-- einen thermoplastischen heißschmelzenden Klebstoff (B*) umfasst,

(d) und Bedecken der Matrixschicht mit der rückseitigen Auflage.

**11.** Verfahren nach Anspruch 10,
wobei der Zwiebelextrakt (A*) aus Schritt (b) durch einen Prozess erhalten werden kann, der die Schritte umfasst:

(a1) Extrahieren frischer oder getrockneter Zwiebeln, vorzugsweise *Allium cepa,* mit einem Lösungsmittel $S_1$, umfassend mindestens einen Alkohol, um eine Flüssigphase $L_1$ und einen festen Rückstand $R_0$ zu ergeben,
(b1) Trennen der Flüssigphase $L_1$ vom festen Rückstand $R_0$,
(c1) Abdampfen der Flüssigphase $L_1$, um einen Rückstand $R_1$ zu ergeben,
(d1) erneutes Lösen des Rückstands $R_1$ in einem Lösungsmittel $S_2$, umfassend mindestens einen Alkohol und Wasser, um den Zwiebelextrakt (A\*) zu ergeben.

12. Verfahren nach Anspruch 10 oder 11,
wobei die Beschichtungsmasse einen thermoplastischen heißschmelzenden Klebstoff umfasst, und wobei der Schritt (a) umfasst:

- Lösen oder Dispergieren der thermoplastischen heißschmelzenden Klebstoffzusammensetzung (B\*\*) in einem Lösungsmittel $S^{hm}$, Mischen dieser Lösung oder Dispersion mit mindestens einem Zwiebelextrakt (A\*), um die Beschichtungsmasse zu ergeben, und Aufbringen der Beschichtungsmasse auf der Abziehauflage.

13. Verfahren nach einem der Ansprüche 10 bis 12,
wobei das Trocknen in Schritt (c) in vier Trocknungszonen mit unterschiedlichen Temperaturen durchgeführt wird, wobei vorzugsweise in der ersten Zone die Temperatur im Bereich von 35°C bis 45°C liegt, in der zweiten Zone die Temperatur im Bereich von 45°C bis 55°C liegt, in der dritten Zone die Temperatur im Bereich von 85°C bis 95°C liegt, und wobei in der vierten Zone die Temperatur im Bereich von 115°C bis 125°C liegt.

14. Verfahren nach Anspruch 12 oder 13,
wobei das Trocknen in Schritt (c) in einer kontinuierlichen Beschichtungslinie mit vier Temperaturzonen durchgeführt wird, und das Trocknen mit einer Bahngeschwindigkeit im Bereich von 1,3 m/min bis 1,8 m/min vorgenommen wird.

15. Pflaster nach einem der Ansprüche 1 bis 9,
welches durch ein Verfahren nach einem der Ansprüche 10 bis 14 erhalten wird oder erhalten werden kann.

16. Pflaster nach einem der Ansprüche 1 bis 9 oder Anspruch 15,
zur Verwendung bei der Behandlung und/oder Prävention einer Narbe.

**Revendications**

1. Patch comprenant une composition en couche, dans lequel la composition en couche comprend au moins :

- une doublure de support,
- une couche de matrice comprenant un extrait d'oignon (A), et

- un polymère à base d'acrylate (B), ou
- un adhésif thermofusible thermoplastique (B\*),

- et une doublure de libération, où

la couche de matrice est disposée entre la doublure de support et la doublure de libération de sorte que la couche de matrice entre en contact avec la doublure de support et la doublure de libération sur des côtés opposés de celle-ci, où la doublure de libération peut être libérée à partir de la couche de matrice.

2. Patch selon la revendication 1, dans lequel la couche de matrice comprend l'extrait d'oignon (A) selon une quantité dans la plage allant de 0,01 à 5% en poids, sur la base du poids total de la couche de matrice et calculée comme l'extrait d'oignon sec.

3. Patch selon les revendications 1 ou 2, dans lequel l'extrait d'oignon (A) est un extrait d'*Allium cepa.*

4. Patch selon l'une quelconque des revendications 1 à 3, dans lequel la couche de matrice comprend un polymère à base d'acrylate (B) et un extrait d'oignon (A), et où le polymère (B) est un polymère à base d'acrylate-acétate de vinyle comprenant des synthons dérivés de composants monomères d'acrylate (B1) et d'acétate de vinyle (B2).

**5.** Patch selon l'une quelconque des revendications 1 à 3, dans lequel la couche de matrice comprend un adhésif thermofusible thermoplastique (B*) et un extrait d'oignon (A), et où le polymère (B*) est un caoutchouc thermoplastique thermofusible, comprenant de préférence un polymère à base de styrène, une résine tackifiante et un plastifiant.

**6.** Patch selon l'une quelconque des revendications 1 à 3 et 5, dans lequel la couche de matrice comprend un adhésif thermofusible thermoplastique (B*) et un extrait d'oignon (A), où la couche de matrice est obtenue par

- la solubilisation ou la dispersion d'une composition d'adhésif thermofusible thermoplastique (B**) dans un solvant S$^{hm}$,
- le mélange de cette solution ou dispersion avec au moins un extrait d'oignon (A*) afin de donner une masse de revêtement,
- l'application de la masse de revêtement sur une doublure de libération, et
- le séchage de la doublure de libération et de la masse de revêtement y étant appliquée, de façon à ce que la couche de matrice comprenant un extrait d'oignon (A) et l'adhésif thermofusible thermoplastique (B*) soit formée sur la couche de libération, et comprenant un extrait d'oignon (A*) et un adhésif thermofusible thermoplastique (B*) sur la doublure de libération.

**7.** Patch selon la revendication 6, dans lequel la composition d'adhésif thermofusible thermoplastique B** n'est pas fondue préalablement à l'application de la masse de revêtement sur la doublure de libération.

**8.** Patch selon l'une quelconque des revendications 1 à 7, dans lequel la couche de matrice comprend en outre de l'allantoïne, préférablement selon une quantité dans la plage allant de 0,01 à 5% en poids, sur la base du poids total de la couche de matrice.

**9.** Patch selon l'une quelconque des revendications 1 à 8, dans lequel la doublure de support est au moins partiellement faite à partir d'un matériau occlusif, et/ou où la doublure de support est une doublure en mousse, préférablement une doublure en mousse comprenant une polyoléfine.

**10.** Méthode de fabrication d'un patch, comprenant les étapes suivantes :

(a) la fourniture de la doublure de libération,
(b) l'application d'une masse de revêtement comprenant un extrait d'oignon (A*), et

- un polymère à base d'acrylate (B), ou
- une composition d'adhésif thermofusible thermoplastique (B**),

à la doublure de libération,
(c) le séchage de la doublure de libération et de la masse de revêtement y étant appliquée, de façon à ce que la couche de matrice soit formée sur la couche de libération, ladite couche de matrice comprenant un extrait d'oignon (A) et

- un polymère à base d'acrylate (B), ou
- un adhésif thermofusible thermoplastique (B*), (d) et le recouvrement de la couche de matrice au moyen de la doublure de support.

**11.** Méthode selon la revendication 10, dans laquelle l'extrait d'oignon (A*) de l'étape (b) peut être obtenu par un procédé comprenant les étapes

(a1) d'extraction d'oignons frais ou séchés, préférablement *Allium cepa,* par un solvant S$_1$ comprenant au moins un alcool afin de donner une phase liquide L$_1$ et un résidu solide R$_o$,
(b1) la séparation de la phase liquide L$_1$ et du résidu solide R$_o$,
(c1) l'évaporation de la phase liquide L$_1$ afin de donner un résidu R$_1$,
(d1) la resolubilisation du résidu R$_1$ dans un solvant S$_2$ comprenant au moins un alcool et de l'eau afin de donner l'extrait d'oignon (A*).

**12.** Méthode selon la revendication 10 ou 11, dans laquelle la masse de revêtement comprend un adhésif thermofusible thermoplastique, et où l'étape (a) comprend

- la solubilisation ou la dispersion de la composition d'adhésif thermofusible thermoplastique (B**) dans un solvant $S^{hm}$, le mélange de cette solution ou dispersion avec au moins un extrait d'oignon (A*) afin de donner la masse de revêtement, et l'application de ladite masse de revêtement sur la doublure de libération.

13. Méthode selon l'une quelconque des revendications 10 à 12, dans laquelle le séchage dans l'étape (c) est effectué dans quatre zones de séchage ayant différentes températures, préférablement où, dans la première zone, la température se trouve dans la plage allant de 35°C à 45°C, dans la deuxième zone, la température se trouve dans la plage allant de 45°C à 55°C, dans la troisième zone, la température se trouve dans la plage allant de 85°C à 95°C, et dans la quatrième zone, la température se trouve dans la plage allant de 115°C à 125°C.

14. Méthode selon la revendication 12 ou 13, dans laquelle le séchage dans l'étape (c) est effectué dans une ligne d'enduction continue ayant quatre zones de température et le séchage est effectué avec une vitesse de la bande dans la plage allant de 1,3 m/min à 1,8 m/min.

15. Patch selon l'une quelconque des revendications 1 à 9, obtenu ou pouvant être obtenu par une méthode selon l'une quelconque des revendications 10 à 14.

16. Patch selon l'une quelconque des revendications 1 à 9 ou selon la revendication 15, pour une utilisation dans le traitement et/ou la prévention d'une cicatrice.

Fig. 1

Fig. 2

Fig. 3

Fig. 4

Fig. 5

Fig. 6

Fig. 7

Fig. 8

Fig. 9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5885581 A
- US 20100247689 A
- WO 2011006100 A
- US 2006127437 A
- DE 3723248 A
- EP 429080 B
- EP 364442 B
- EP 201956 B
- US 5759560 A
- US 5891076 A
- US 5895656 A
- US 5919476 A
- EP 663431 A
- EP 452034 A
- EP 305757 A
- DE 310012 A4
- DE 4222334 A
- DE 4224325 C